Europäisches Patentamt
European Patent Office
Office européen des brevets

(19) (11) Publication number: **0 072 872 A1**

(12) **EUROPEAN PATENT APPLICATION**

(21) Application number: 81106499.7

(22) Date of filing: 21.08.81

(51) Int. Cl.³: C 07 C 177/00
A 61 K 31/557
//C07C59/245, C07D317/34,
C07D307/54, C07F7/18,
C07D317/22, C07D317/16,
C07F9/54, C07D307/30,
C07C49/203

(43) Date of publication of application:
02.03.83 Bulletin 83/9

(84) Designated Contracting States:
DE FR GB


(71) Applicant: AMERICAN CYANAMID COMPANY
1937 West Main Street P.O. Box 60
Stamford Connecticut 06904(US)

(72) Inventor: Wissner, Allan
31 Wood Avenue
Ardsley New York(US)

(72) Inventor: Floyd, Middleton Brawner, Jr.
5 Babbling Brook Lane
Suffern New York(US)

(74) Representative: Wächtershäuser, Günter, Dr.
Tal 29
D-8000 München 2(DE)


(54) **1-Hydroxymethyl-prosten-1-ol derivatives.**


(57) There is provided novel 1-hydroxy-1-hydroxymethyl prostaglandins and method of use for vasodepression, smooth muscle stimulation and as antilipolytic agents. The novel compounds have the structure:

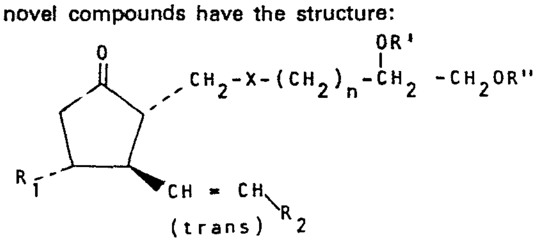

wherein n is the interger 3-5 inclusive; X is $CH_2CH_2$ or CH = CH (cis); $R_1$ is hydrogen or hydroxy, R' and R'' are the same or different and are hydrogen, $C_1$ to $C_4$ alkyl, $C_1$ to $C_6$ alkanoyl or optionally substituted benzoyl, wherein said substituents on said benzoyl moiety are selected from the group $C_1$ to $C_4$ alkoxy, halo and trifluoromethyl; and $R_2$ is selected from the group consisting of


./...

EP 0 072 872 A1

Croydon Printing Company Ltd

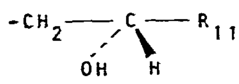, 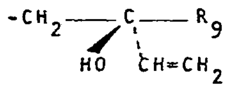, 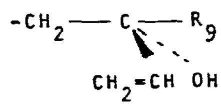

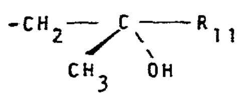, 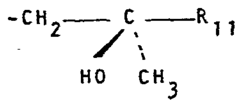, 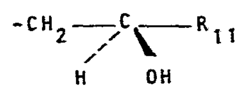

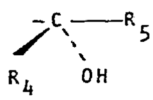 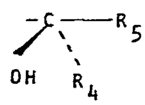

wherein $R_4$ is hydrogen or methyl; $R_5$ is selected from the group consisting of $C_4$-$C_7$ alkyl; $R_9$ is selected from the group consisting of $C_3$-$C_6$; $R_{11}$ is selected from the group consisting of $C_3$-$C_7$ alkyl; the racemic mixtures thereof and the individual optically active enantiomers thereof; and the pharmaceutially acceptable non-toxic salts thereof.

# 1-HYDROXYMETHYL-PROSTEN-1-OL DERIVATIVES

The present invention relates to 1-hydroxy-1-hydroxymethyl prostaglandins, as well as the pharmaceutically acceptable, non-toxic $C_1$ to $C_4$ alkyl esters thereof, and to the intermediates and processes for producing such compounds.

Prostaglandins have classically been described as chemically related 20 carbon chain hydroxy fatty acids having the basic skeleton of prostanoic acid:

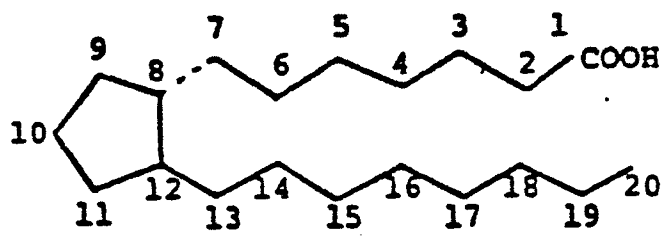

The prostaglandins having a hydroxyl group at the C-11 position and a keto group at the C-9 position are known as the PGE series, and those having a hydroxyl group in place of the keto group are known as the PGF series and are further designated by an  or B suffix to indicate the configuration of the hydroxyl group at said position. The natural compounds are the α-hydroxy substituted compounds. They may contain different degrees

of unsaturation in the molecule, particularlyat C-5, C-13 and C-17, the unsaturation is also indicated by a suffix. Thus, for example, the $PGF_1$ and $PGE_1$ series refer to prostanoic acids having a trans olefin bond at the C-13 position, while the $PGF_2$ and $PGE_2$ series refer to prostadienoic acids having a cis-olefin bond at the C-5 position and a trans olefin bond at the C-13 position. For a review on prostaglandins and the definition of primary prostaglandins, see, for example, P. Ramwell, The Prostaglandins, 1, pp. 5-22 (1973).

The preparation of derivatives of prostanoic acid has become of great importance since the demonstration of the highly interesting range of biological and pharmacological activities of natural prostaglandins.

The great majority of these studies have focused on modification of the two side chains, or modifications substitutents attached to the cyclopentenone moiety [see for example U. Axen et al., Synthesis Vol. 1, John Wiley and Sons Inc., New York, N.Y. 1973 and P. H. Bently, Chem. Soc. Reviews 2, 29 (1973)].

The synthesis of prostaglandin analogs possessing a 3-oxa- or 11-deoxy-3-thia moiety have been described, among others in U.S. Patent No. 3,873,607; U.S. Patent No. 3,950,406; Netherlands Patent 7305222-Q; U.S. Patent No. 3,944,593; U.S. Patent No. 3,931,289; and U.S. Patent No. 3,936,487.

The synthesis of several prostaglandin analogs wherein the hydroxyl group at C-15 has been removed and a hydroxyl group has been introduced at C-16 has appeared [see, for example, U.S. Patent No. 3,950,406; Prostaglandins, Vol. 10, 733 (1975); Tetrahedron Letters, No. 48, 4217 (1975)].

Recently reports have also appeared wherein the C-16 carbon bearing a hydroxyl group is substituted with a methyl group [see Pappo *et al.*, Tetrahedron Letters, No. 4, 235 (1975); Collin *et al.*, U.S. Patent No. 3,965,143; and Belgium Patent No. 827,127].

Also, a patent has recently appeared wherein the C-16 carbon bearing the hydroxyl group is substituted with vinyl, methylvinyl, and cyclopropyl (U.S. Patent 4,061,670).

The present invention is directed to the compounds of the formula:

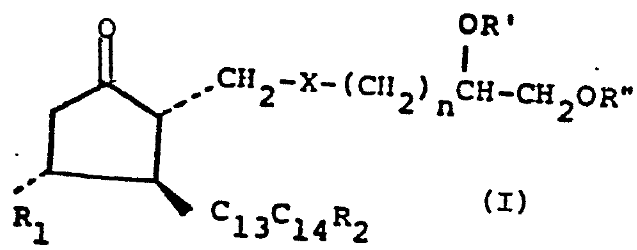

(I)

wherein n is the interger 2-5 inclusive; x is $CH_2CH_2$ or -CH = CH-(cis); $R_1$ is hydrogen or hydroxy; R' and R" are the same or different and are hydrogen, $C_1$ to $C_4$ alkyl, $C_1$ to $C_6$ alkanoyl or optionally substituted benzoyl, wherein said substituents on said benzoyl moiety are

selected from the group $C_1$ to $C_4$ alkoxy, halo and trifluoromethyl; $C_{13}$-$C_{14}$ is $-CH_2CH_2$ or -CH=CH-(trans) and $R_2$ is selected from the group consisting of:

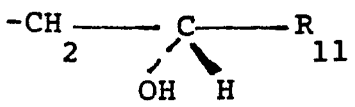, 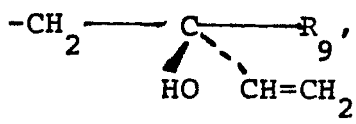, 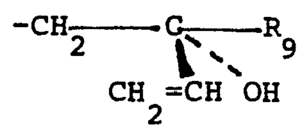

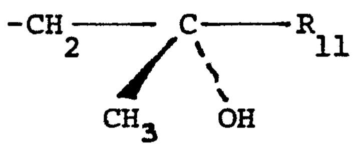, 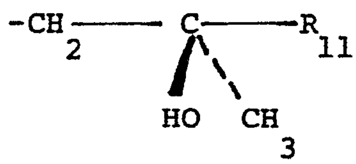, 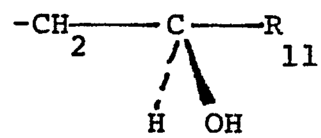

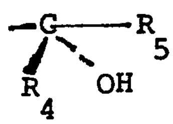 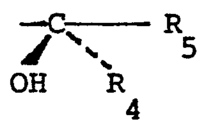

wherein $R_4$ is hydrogen, or methyl; $R_5$ is selected from the group consisting of $C_4$-$C_7$ alkyl; $R_9$ is selected from the group consisting of $C_3$-$C_6$; $R_{11}$ is selected from the group consisting of $C_3$-$C_7$ alkyl; the racemic mixtures thereof and the individual optically active enantiomers thereof; and the pharmaceutically acceptable non-toxic salts thereof.

In particular, the compounds of this invention can be prepared by a 1,4-conjugate-addition procedure involving treatment of an ether blocked cyclopentenone such as (64) or (70) with a lithio-cuprate reagent such as (74), (75), or (76), prepared as illustrated in Flowsheets A through G which follow.

The 1,4 conjugate-addition procedure is described hereinbelow. The preparation of the various requisite 1-iodo-*trans*-1-alkenyl or 1-tributylstannyl-*trans*-1-alkenyl derivatives is illustrated in Flowsheets A-C and the methods of preparation of the 4-hydroxycyclopentenones embracing the 1-(hydroxymethyl)-1-hydroxy chain are described in connection

with Flowsheets D and E.

In accordance with the procedure as outlined in Flowsheet A, an aldehyde (34) is treated with propargylic magnesium halide to form the homopropargylic alcohol (35), which is converted to its trimethylsilyl ether in the usual manner. The silylated derivative is then treated with disiamylborane (prepared *in situ* in tetrahydrofuran solution at ice bath temperature from 2-methyl-2-butene, sodium borohydride and boron trifluoride ethereate) and then anhydrous trimethylamine oxide. The resulting solution and an iodine solution in tetrahydrofuran are added simultaneously to a sodium hydroxide solution to give the 1-iodo-4-trimethylsilyloxy-*trans*-1-alkene (36), precursors for 16-hydroxy-prostaglandins.

The trimethylsilyl protecting group is removed with mild acid and the resulting vinyl iodide alcohol is oxidized with pyridinium chlorochromate to provide the 1-iodo-4-oxo-*trans*-1-alkene (37), which upon treatment with a Grignard reagent ($R'_{13}MgX$) provides the 1-iodo-4-hydroxy-*trans*-1-alkene, which is silylated in the usual manner to provide the silyl either (38) wherein $R'_{11}$ is a $C_3$ to $C_7$ alkyl or an alkenyl group and $R'_{13}$ is vinyl, methyl, or hydrogen.

FLOWSHEET A

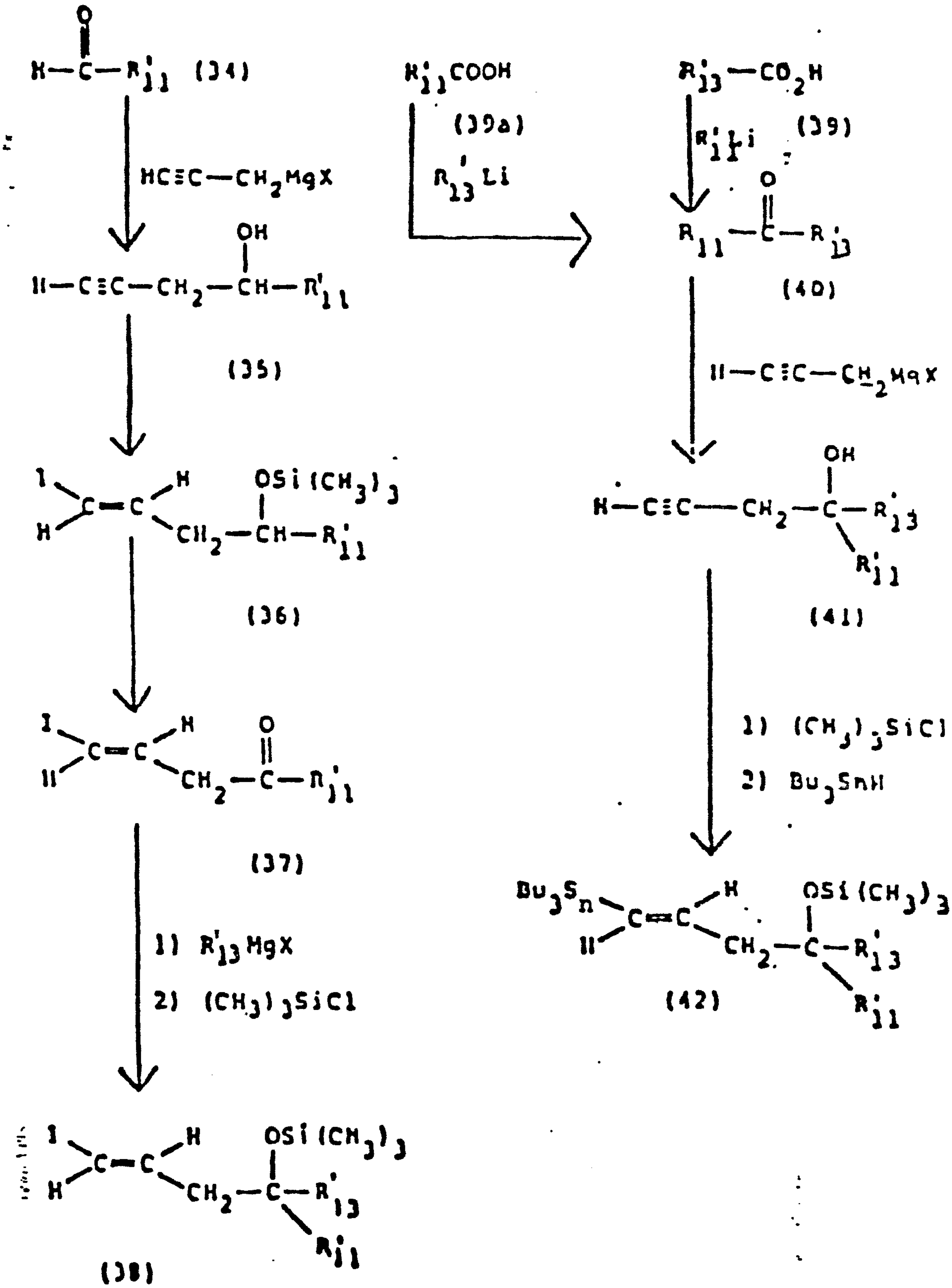
(34)
(39a)
(39)
$HC\equiv C-CH_2MgX$
$R'_{13}Li$
$R'_{11}Li$
(40)
(35)
$H-C\equiv C-CH_2MgX$
(36)
(41)
(37)
1) $(CH_3)_3SiCl$
2) $Bu_3SnH$
1) $R'_{13}MgX$
2) $(CH_3)_3SiCl$
(42)
(38)

A preferred method for the preparation of vinyl-lithium precursor is also described in Flowsheet A. Treatment of the requisite carboxylic acid (39 or 39a) with the appropriate organolithium reagent ($R'_{13}Li$ or $R'_{11}Li$ respectively), wherein $R'_{11}$ and $R'_{13}$ are hereinabove defined, give the corresponding ketone (40) which upon treatment with propargylic magnesium halide provides the homopropargylic alcohol (41) which is converted to the trans vinylstannyl derivative by sequential treatment with chlorotrimethylsilane and tri-n-butyltin hydride. Treatment of the vinylstannyl reagent (42) with n-butyllithium at a temperature of -10°C to 78°C generates the corresponding vinyllithium reagent.

FLOWSHEET B

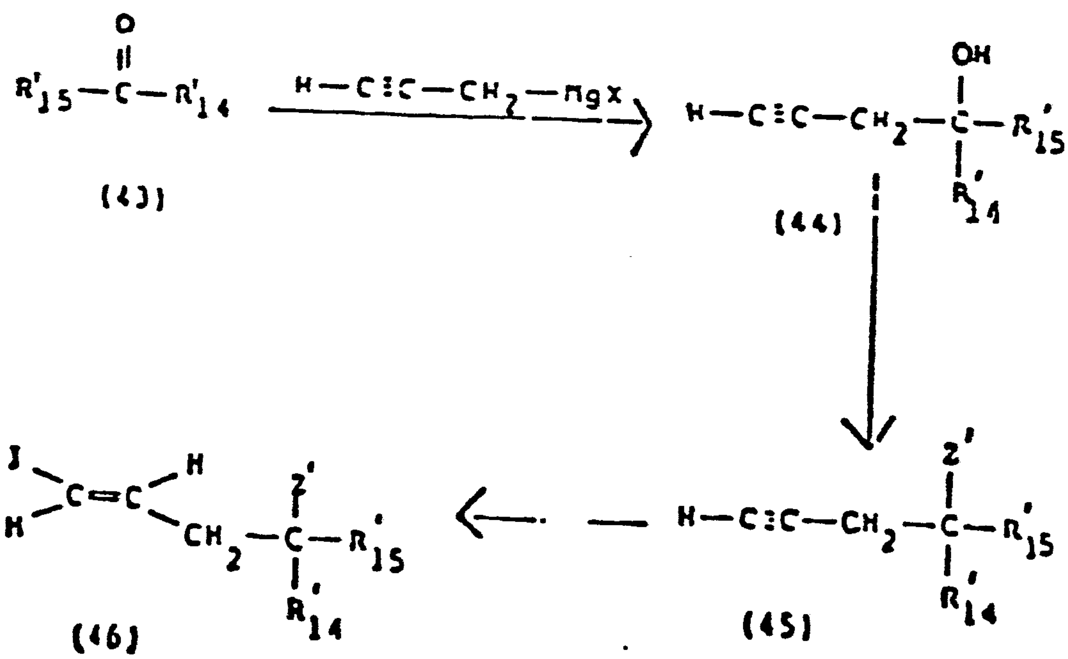


(43)

(44)

(45)

(46)

In accordance with Flowsheet B hereinabove, the precursors for 16-hydroxy prostaglandins are prepared by treating an appropriate aldehyde or ketone (43) with a propargylic magnesium halide to yield the requisite homopropargylic alcohol (44). The alcohol is protected as a trityl-ether (45) (for secondary alcohols) or as a trimethylsilyl ether (45) (for tertiary alcohols). These ethers are then converted to the appropriate trans-vinyliodide (46) by treatment with disiamylborane generated in situ from 2-methyl-2-butene, sodium borohydride, and then iodine and sodium hydroxide, wherein $R'_{15}$ is hydrogen, or methyl; Z' is $-O-C(C_6H_5)_3$ when $R'_{15}$ is hydrogen and Z' is $-O-Si(CH_3)_3$ when $R'_{15}$ is methyl or ethyl; $R'_{14}$ is selected from the group $C_3$ to $C_7$ alkyl.

The preparation of the precursors for the synthesis of secondary 15-hydroxy congeners and 16-hydroxy congeners are described in the literature. The preparation of the precursor for 15-methyl-15-hydroxy is described in Flowsheet C hereinbelow. See, for example, U.S. Patent No. 4,235,797 which is incorporated herein by reference. In accordance with Flowsheet C, an acid chloride, wherein $R_5$ is a $C_4$-$C_7$ alkyl group, is treated with acetylene and aluminum trichloride to provide the vinylchloride (55) which upon treatment with sodium iodide furnishes the vinyliodide (56). Treatment of (56) with methylmagnesium

halide followed by chlorotrimethylsilane gives the requisite protected vinyliodide (57).

FLOWSHEET C

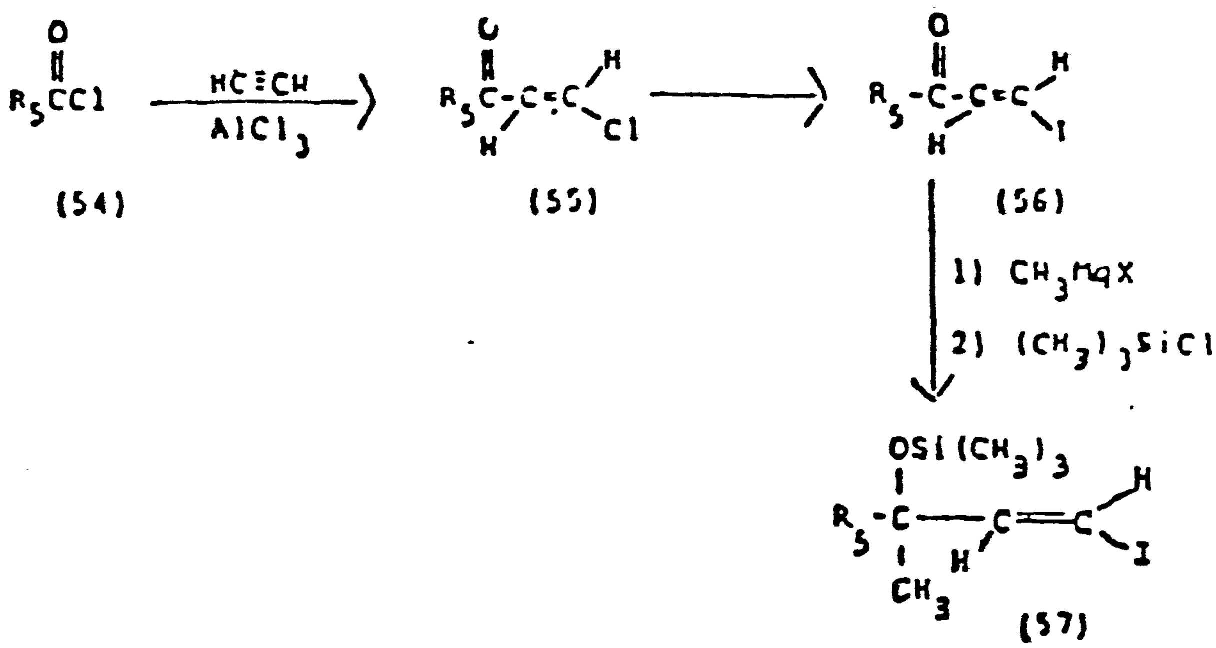


(54) (55) (56) (57)

The procedures set forth above for the preparation of the various vinyl iodide and vinyl tin intermediates have been disclosed with particularity in the following: U.S. Patent No. 4,233,231 and U.S. Patent No. 4,254,145.

The preparation of the cyclopentenones useful in this invention containing the 1-hydroxy-1-hydroxymethyl feature (58a and 59a) where $R_1$ is hydrogen or a hydroxy group can be accomplished in several ways.

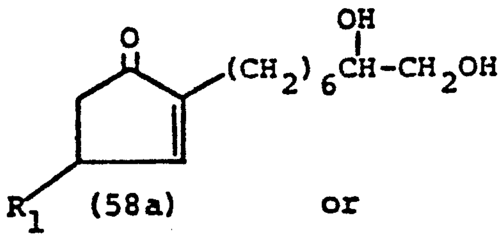

(58a)

or

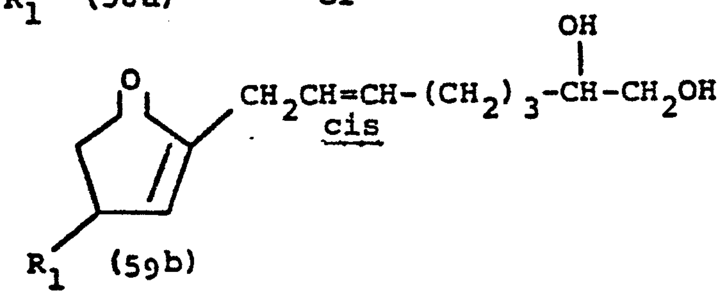

(59b)

The preparation of the respective hydroxy-hydroxymethyl analogs and the protection of these compounds for a conjugate addition reaction is described hereinbelow in Flowsheets D and E.

For the preparation of cyclopentenones of the type wherein the intermediate includes the group CH=CH-(cis), as shown by structure 59b. An alkyl vicinal diol-substituted triarylphosphonium halide is first prepared by reaction of 4-(ω-halo $C_4$ to $C_6$ alkyl)-2,2,-dimethyl-1,3-dioxolane with triphenylphosphine in the presence of a polar inert solvent, for example, dimethyl-formamide, acetonitrile, etc. sometimes admixed with acetone. (See Flowsheet D).

The reaction is typically conducted at elevated temperatures (60°-100°) for from about 24 to 178 hours. The alkyldiol-substituted triphenylphoshonium halide (60) obtained is then used in a subsequent Wittig Reaction with a 2,5-dihydro-2,5- $C_1$ to $C_4$ alkoxy-2[3-(1-oxo) propyl] furan (61) to produce a 2,5-dihydro-2,5-di $C_1$ to $C_4$ alkoxy-2-(ω,ω-1-dihydroxy-3-$C_8$- to $C_{10}$ alkenyl) furan (62). The reaction is carried out in demethylsulfoxide typically at 0° to 50° in the presence of the Wittig reagent, prepared by treating dimsyl sodium with compound (60) typically in the same inert solvent. Conversion of compound (62) to the required cyclopentenones (63) is effected by sequential treatment of (62) with wet silica gel and, after isolation of crude intermediate material, with aqueous mineral acid in a co-solvent such as dioxane or dimethoxyethane. A preferred system is one normal sulfuric acid in aqueous dioxane at reflux temperatures for a period of 24 hours.

Protection of the hydroxy groups of compounds (63) suitable for the conjugate addition reaction is accomplished by treating these compounds with hexa $C_1$ to $C_4$ alkyl disilazane and a halo-tri-$C_1$ to $C_4$ alkylsilane such as chlorotimethylsilane. The components are admixed in a suitable polar organic solvent, i.e., pyridine for from about 1 to about 24 hours at 0° to 75° affording the compounds (64).

The preparation of cyclopentenones of the type used in the preparation of the compounds of formula 58a is accomplished by reaction of a 2-hydroxy suberic, azelic or sebacic acid (65) and acetone to yield the corresponding 4-(ω-carboxy $C_5$ to $C_7$ alkyl)-2,2-dimethyl-5-oxo-1,3-dioxolane (66). See Flowsheet E. The cyclization is carried out in the presence of catalytic amounts of an acid catalyst, i.e., sulfuric acid, hydrochloric acid, p-toluenesulfonic acid and the like, typically an inert organic solvent such as acetone, petroleum ether or mixtures thereof for a time and at a temperature sufficient to complete the reaction. Preferably the reaction is conducted under reflux conditions with the simultaneous azeotropic removal of water until no additional water accumulates in the distillate. Compounds (66) are converted, through the intermediacy of their mixed anhydrides with trifluoroacetic acid followed by Fridel-Crafts acylation of furan, into ω-(2-furoyl)-2-hydroxy $C_7$ to $C_9$ alkanoic acids (67). Typically the reaction is accomplished in a polar inert organic solvent, i.e., chloroform, methylene dichloride, etc., at 0° to 50° for 5 minutes to 40 hours. Compounds (67) are selectively reduced to compounds (68) using borane-tetrahydorfuran complex at temperatures of -10° to 20° over 1 to 24 hours. Solvents of preference include the linear and cyclic ethers

such as diethyl ether, tetrahydrofuran, and the like.

Rearrangement of compounds (68) to the ω-(4-hydroxy-cyclopent-2-en-1-on-2-yl)alkane-1,2 diols (69) is accomplished by: (1) Treatment of (68) with a solution of weak aqueous acid in a solvent such as dioxane or dimethoxyethane at a temperature of 75°-100°. A preferred system is two normal formic acid in aquous dioxane at reflux temperatures for a period of 24 hours; (2) after completion of the first stage, the reaction solution is treated with a strong acid, preferably sulfuric acid in a quantity sufficient to make the solution one normal, and the resulting reaction solution is further heated to effect final conversion to (69). A preferred set of conditions employs the above-prepared solution one normal in sulfuric acid in aqueous dioxane at reflux temperatures for a period of 24 hours.

FLOWSHEET D

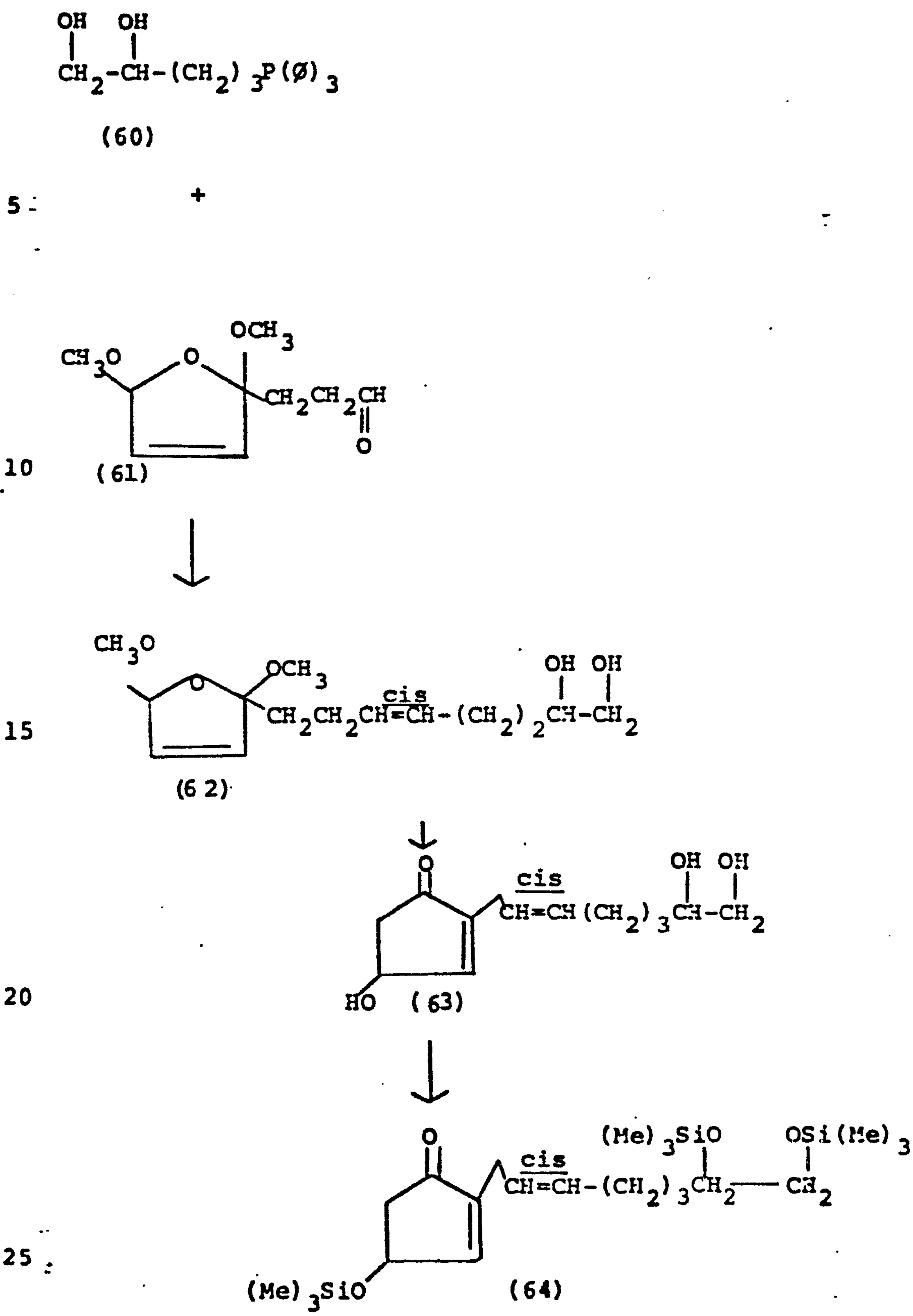

(60)

(61)

(6 2)

(63)

(64)

FLOWSHEET E

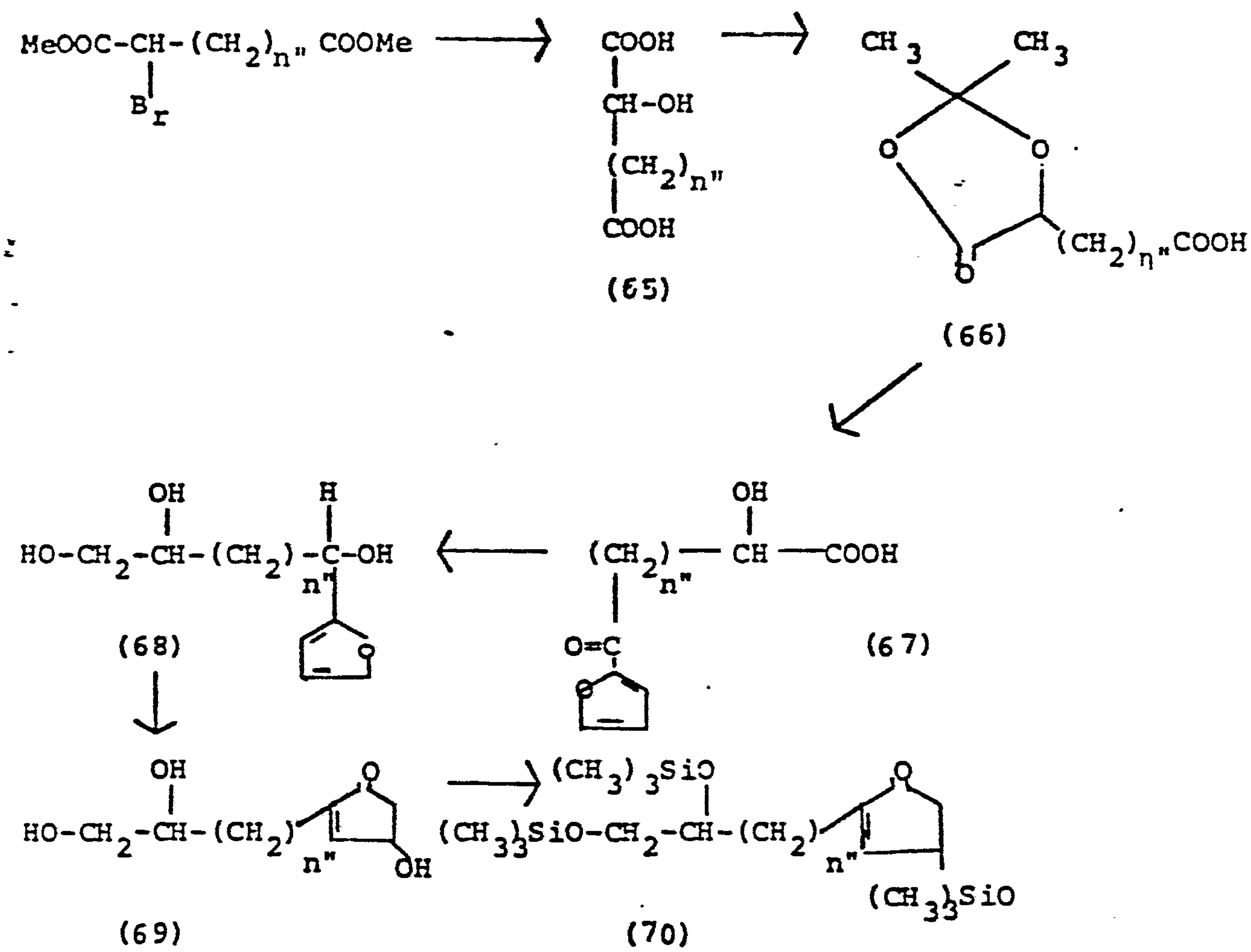

(65) (66) (67) (68) (69) (70)

wherein n" = 6 to 8.

The hydroxy groups of compounds (69) may also be protected using the procedure described for compounds (63), which gives rise to compounds (70).

The preparation of the compounds of formula I from the requisite intermediate cyclopentenones (the preparation of such of which being disclosed above) is accomplished by a conjugate addition reaction using organocopper reagents. These procedures are well known in the art and are described in, for example, C.J. Sih, et al J. Amer. Chem. Soc., 97 865 (1978). A brief description of the reaction sequence is set forth below and in the Preparations and Examples herein.

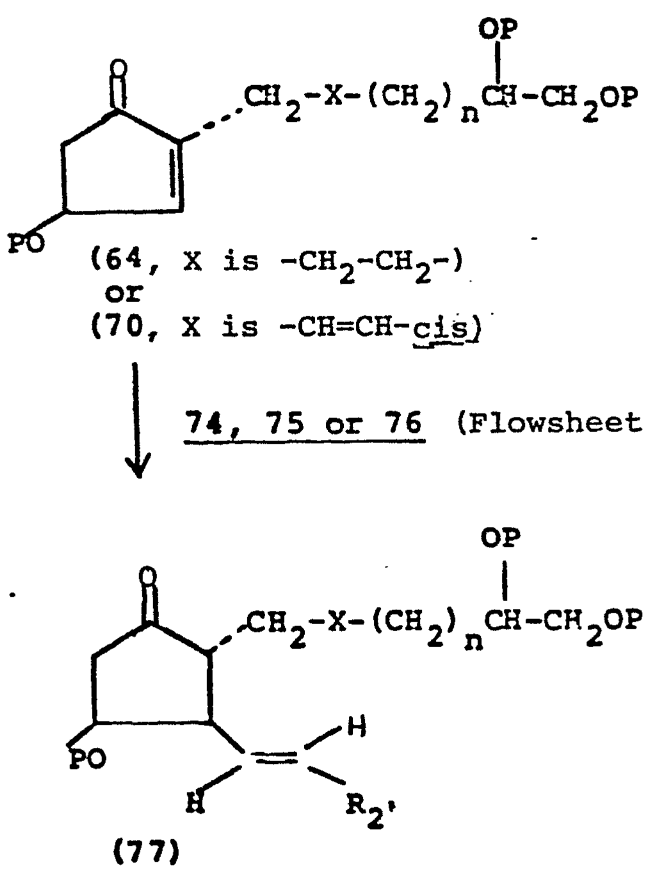


(64, X is $-CH_2-CH_2-$)
or
(70, X is $-CH=CH-$*cis*)

(77)

$R_2'$ represents an $R_2$ group wherein the hydroxyl substituents of this moiety have been protected by the procedures shown above in Flowsheets A through C, P is a protecting group and $R_2$ and X are as previously defined. The protecting groups P are selected from any of the protecting groups well known to those skilled in this art and include the groups tri-$C_1$ to $C_4$ alkylsilyl, such as those obtained from the silylchlorides; triphenylmethyl, such as those obtained from the trityl halides; p-methoxy triphenylmethyl, from p-methoxytriphylmethyl halides; methoxymethyl, from chloromethylether; as well as those protecting groups obtained from 2-methoxypropene; dihydro-2H-pyran, ethylvinylithio and the like. Typically the hydroxy compounds of formulae 69 and 63 are heated with an amount of the protecting group reagent, in sufficient excess to assure complete reaction of each hydroxy moiety, i.e., 2 equivalents of protecting group reagent per equivalent of hydroxy moiety, in the absence or presence of an inert organic solvent.

In accordance with Flowsheet F the vinyliodide (71) is treated with either one equivalent of n-butyllithium or 2 equivalents of t-butyllithium at low temperature, preferably -30° to -70° in an inert solvent, e.g., hexane, ether or toluene to provide the trans alkenyllithium reagent (73).

Alternatively, the vinyllithium reagent (73) can be prepared by treatment of a vinylstannyl derivative such as (72) with n-butyllithium at -10° to -78° in ether or THF.

For the preparation of the asymmetrical lithio cuprate (74) or the like, a solution of one molar equivalent of copper (I)-1-alkyne, preferably one to five molar equivalents, and anhydrous ether is added to one molar equivalent of the aforementioned vinyllithium solution cooled to about -78°C. After about one hour at this temperature, a molar equivalent of the requisite cyclopentenone (64) or (70) is added. After several hours at -78°C to 20°C the reaction mixture is quenched with aqueous ammonium chloride solution and the blocked product (77) is isolated in the usual manner (and see Flowsheet G).

It is also possible to effect conjugate 1,4-addition with the asymmetrical lithio cuprate (76) derived from vinyllithium (73) and cuprous thiophenoxide. A solution of vinyllithium (73) in ether at -78°C is reacted with equimolar amounts of a reagent prepared by admixture in ether at 0°C to -78°C of equimolar amounts of cuprous thiophenoxide and copper (I) iodidetributylphosphonium complex. After about 30 minutes at this temperature, the lithio cuprate (76) is treated with the requisite cyclopentenone (64) or (70) as described hereinabove for the conjugate addition with 1-alkynyl lithio cuprate (74).

For the preparation of the symmetrical lithio cuprate (75) one molar equivalent of copper (I) iodide-tributylphosphine complex, dissolved in anhydrous ether, is added at about -78°C to two molar equivalents of the aforementioned vinyllithium (73) solution in hexane, cooled to -78°C. After about one hour at this temperature, the lithio cuprate (75) is treated with the requisite cyclopentenone (64) or (70) as described hereinabove for the conjugate addition with the 1-alkynyl lithio cuprate (74). Removal of the blocking groups from (77) to give the prostaglandin congeners of formula I is accomplished by treatment of (77) with a mixture of acetic acid, tetrahydrofuran and water (4:2:1) at 25°C to 55°C. Flowsheet G particularly illustrates these procedures where compounds (87)a and (87(b) are deblocked to yield the compounds of formula (I)a and (I)b. In Flowsheet G, P represents a protecting group, and X, $R_9$, $R_{11}$ and n are as previously defined

FLOWSHEET F

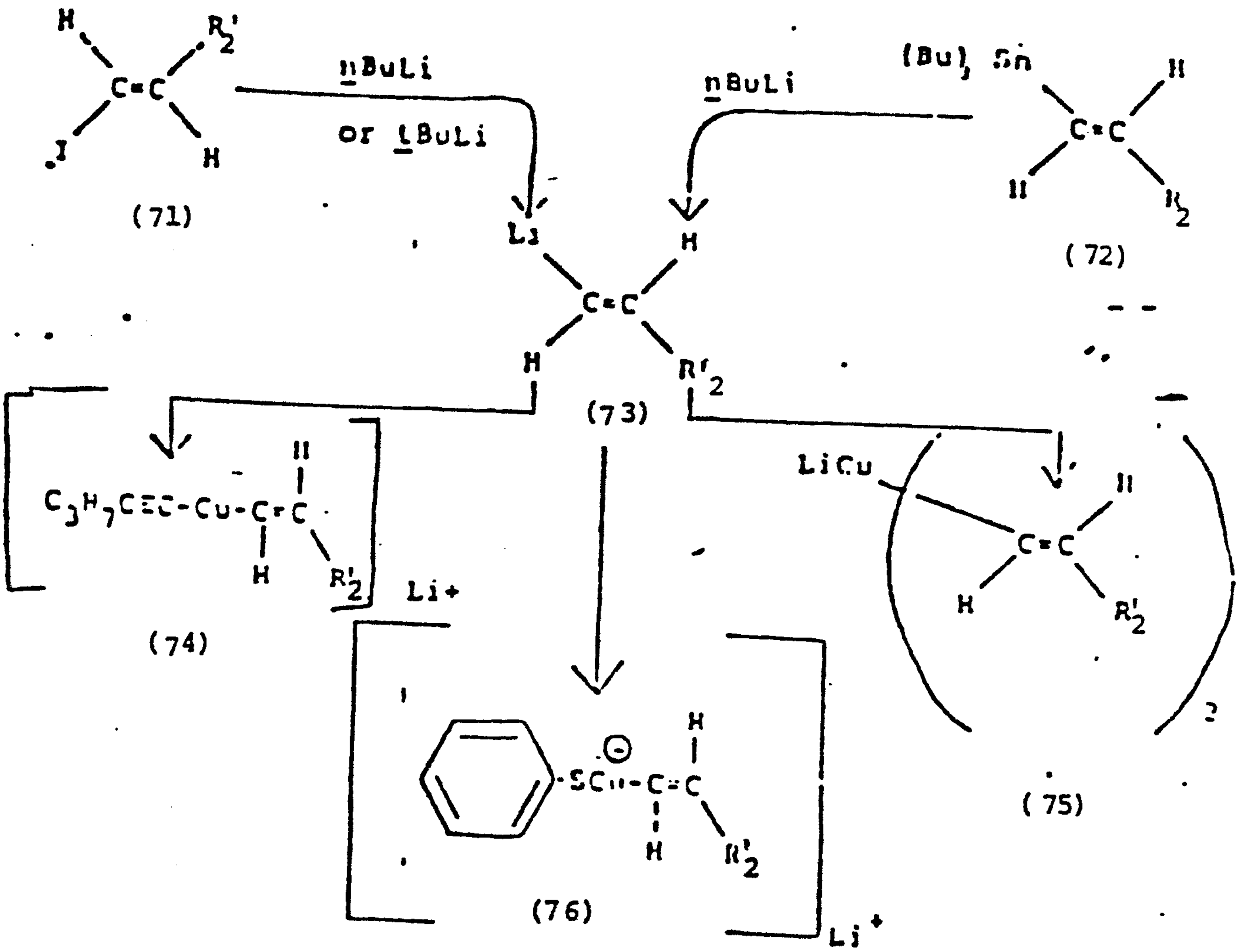


(71) (72) (73) (74) (75) (76)

FLOWSHEET G

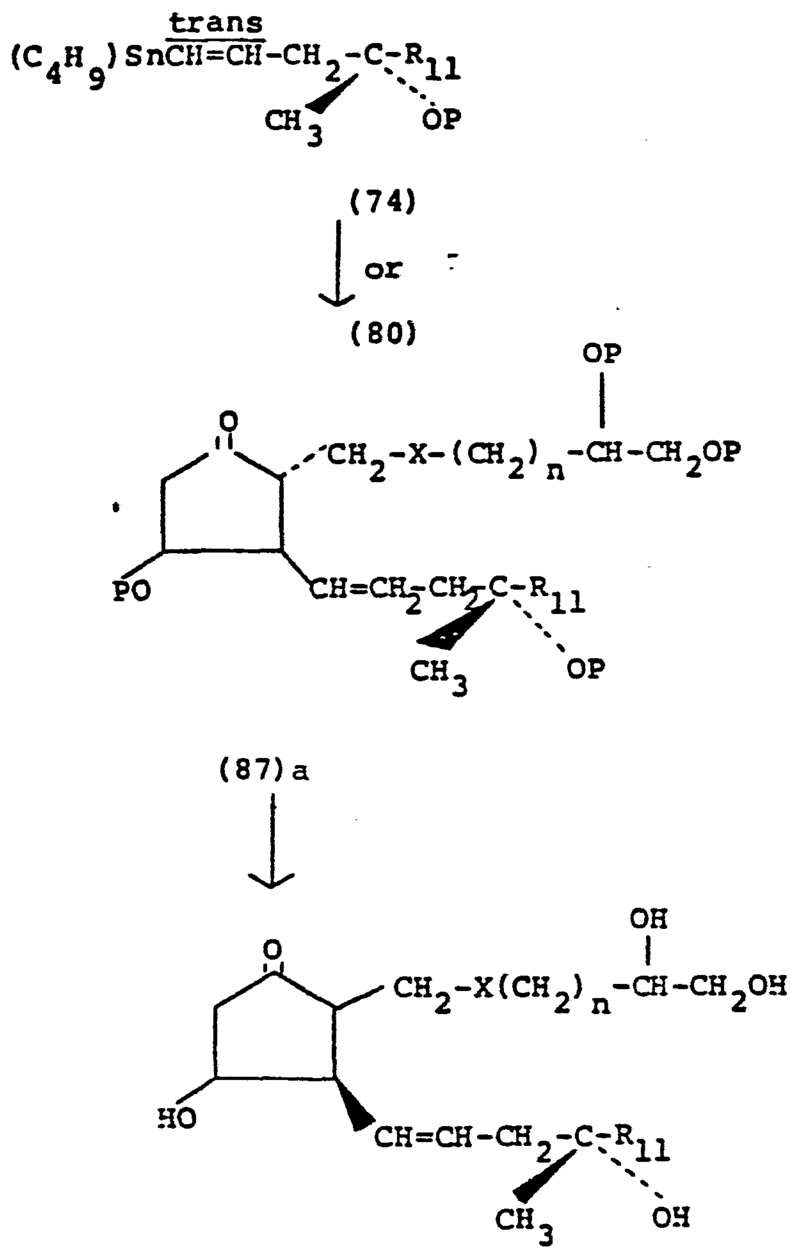


I(a)

FLOWSHEET G CONTINUED

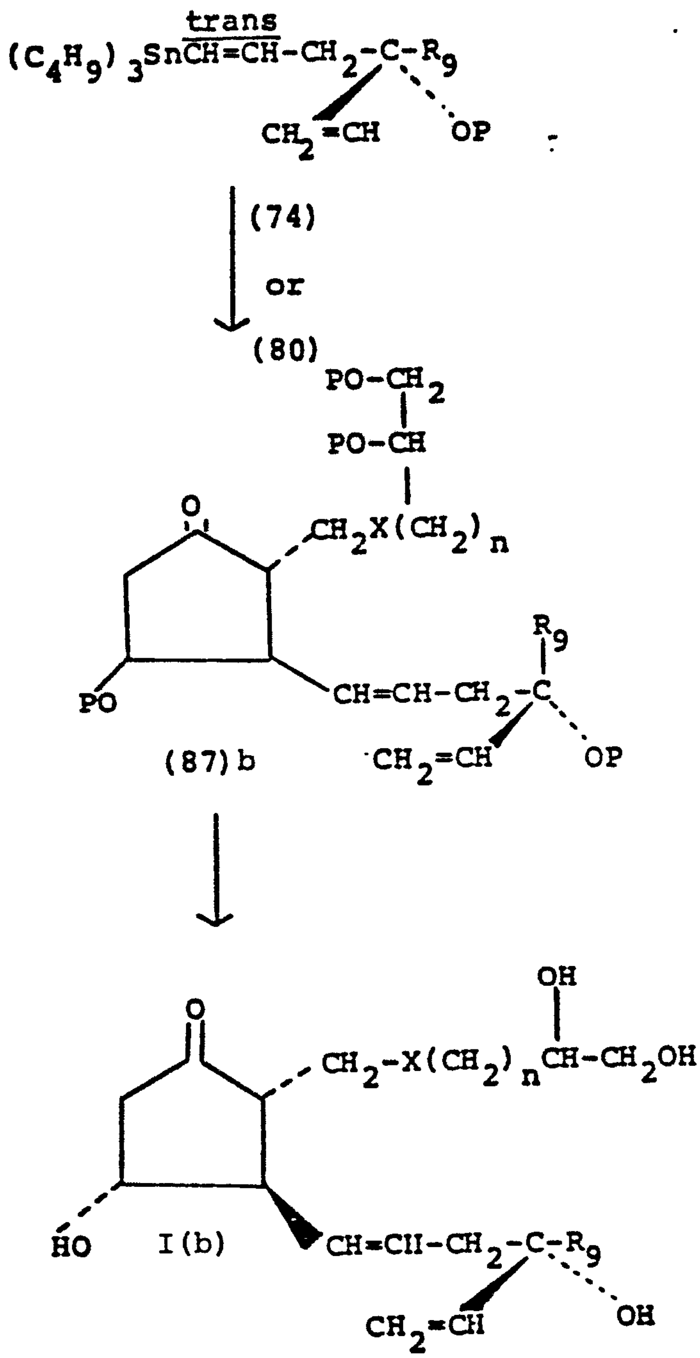


(87)b

I(b)

All available evidence indicates that the $-CH{=}CH-R_2$ function introduced by the cuprate process occupies a position trans to the 11-hydroxy function. Similarly, in the product (e.g., (87)a and (87)b) the two side-chains attached to $C_8$ and $C_{12}$ are trans to each other. However, the configurational relationship in the product as it is obtained directly from the cuprate process may be either cis or trans, i.e., these products may have the side-chains in a trans- or cis-relationship or they may be a mixture containing both the trans- and cis-isomers. This is indicated in the nomenclature of the compounds involved by the designation $\xi$. In order to ensure a trans-relationship in (77) these products can be submitted to conditions known in the literature to equilibrate the cis-8-iso-$PGE_1$ to a mixture containing about 90% of the trans product. These conditions involve treatment with potassium acetate in aqueous methanol for 96 hours at room temperature.

When the compounds of this invention are prepared from racemic starting compounds two racemates are obtained. In appropriate instances these racemates can be separated from each other by careful application of the usual chromatographic procedures. In the more difficult instances it may be necessary to apply high pressure liquid chromatography including recycling techniques. [See G. Fallick,

American Laboratory, 19-27 (August, 1973) as well as references cited therein. Additional information concerning high speed liquid chromatography and the instruments necessary for its application is available from Waters Associate, Inc. Maple Street, Milford, Mass.]

It is also possible to prepare the compounds of this invention in their optically active forms by the conversion of the optically active 2-(ω,ω-1-alkyldiol)-4-hydroxycyclopent-2-en-1-ones (64) or (70) to the optically active protected analogs by procedure well known to the art. The compounds of formula I can be selectively esterified by dissolving the free hydroxy compounds in pyridine and adding one equivalent of an anhydride $(R_{15}-CO)_2O$ or the acid chloride $R_{15}COCl$ and allowing the mixture to stand overnight to give the desired esters (78) and (79).

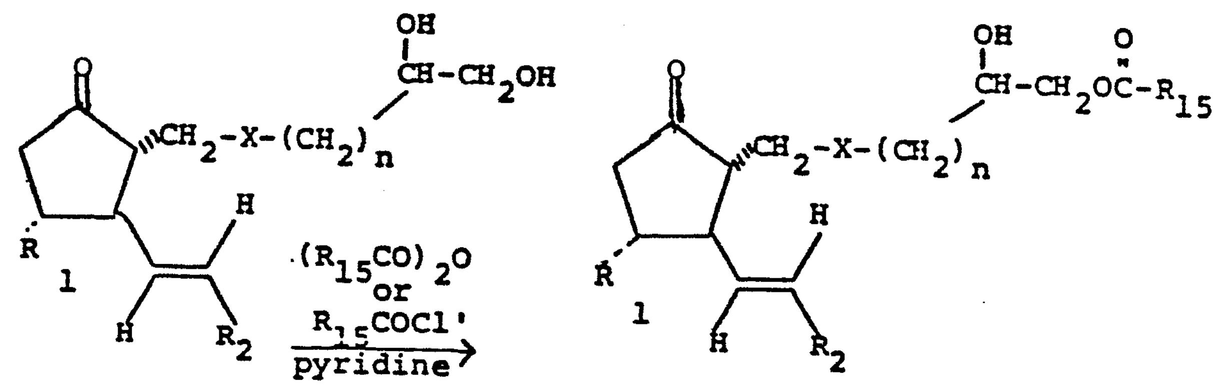


(78)

and

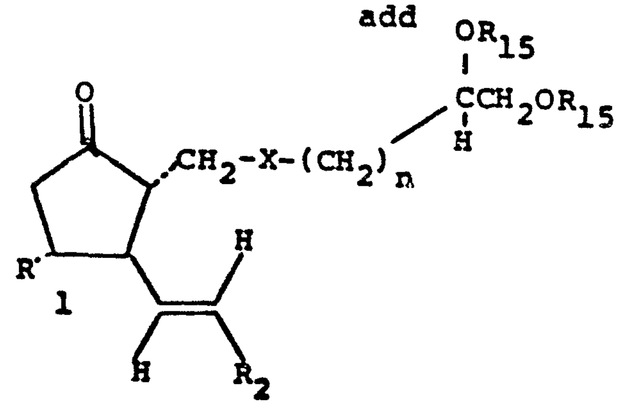

wherein $R_{15}$ is phenyl substituted with one or more groups such as alkyl ($C_1$-$C_4$), OR(R=$C_2$-$C_4$alkyl), SR(R=$C_2$-$C_4$alkyl), F, Cl, dialkylamino, or $C_2$-$C_4$ alkyl.

The 4-hydroxycyclopentenone racemates may be resolved into their component enantiomers (83) and (84) wherein n, X, R' and R" are as hereinabove defined by derivatizing the ketone function with a reagent having an optically active center. The resulting diastereoisomeric mixture can then be separated by fractional crystallization, or by chromatography, or by high speed liquid chromatography involving, if necessary,recycling techniques. Among the useful optically active ketone derivatizing reagents are 1-α-aminoxy-α-methylpentanoic acid hydrochloride (to give (85)), (R)-2-aminoxy-3, 3-dimethylbutyric acid hydrochloride, and 4-α-methylbenzyl semicarbazide. After separation of the distereomeric derivatives, reconstitution of the keto function provides the individual 4-hydroxycyclopentenone enantiomers (83) and (84). A useful procedure for the resolution of a 4-hydroxycyclopentenone racemate via an oxime is described in the art [R. Pappo, P. Collins and C. Jung, Tetrahedron Letters, 943 (1973)]. The resolution of the hydroxycyclopentenone (83) wherein $-CH_2-X(CH_2)_n-$ is 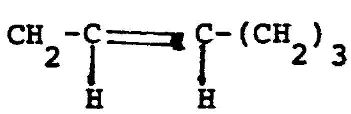 is described by Bruhn *et al.*, *Tetra-*

hedron Letters, 235 (1976).

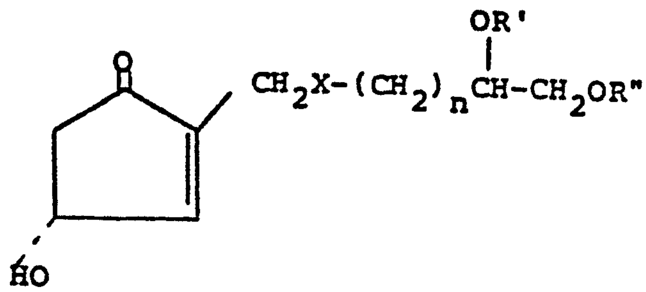

(83)

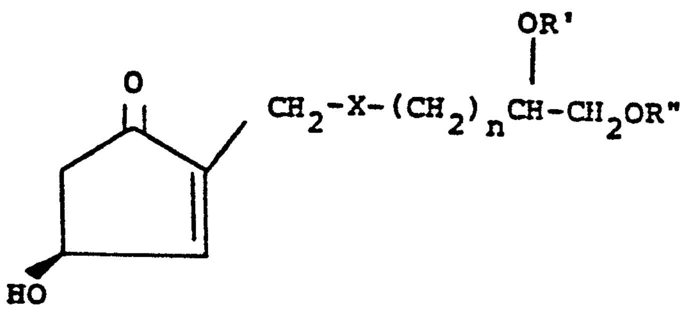

(84)

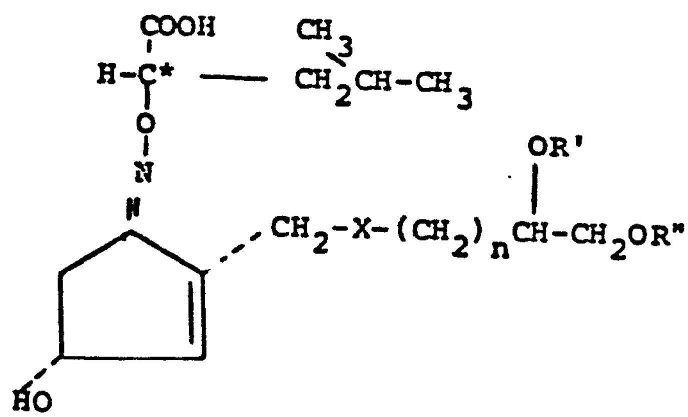

(85)

Other useful ketone derivatizing agents are optically active 1,2-glycols, e.g., D(-)-2,3-butanediol, or 1,2-dithiols, e.g., L(+)-2,3-butanedithiol. These are used to convert the 9-oxo derivative to 9,9-alkylenedioxa or 9,9-alkylenedithia derivatives, separation of diastereomers by chromatographic procedures followed by regeneration of the individual 9-oxo diastereomer by ketal cleavage all by procedures well-known in the art. Both ketalization and deketalization would have to be accomplished by procedures which would not disrupt the 11-oxo-9-keto system, which of course, is not a problem in the 11-unsubstituted series.

An alternate procedure for the preparation of the 4(R)-hydroxycyclopentenone enantiomers such as (83) involves as a key step the selective microbiological or chemical reduction of trione (86) to the 4(R)-hydroxycyclopentanedione (87). A wide variety of microorganisms are capable of accomplishing this asymmetric reduction, one of the most useful being Dipodascus unincleatus. This step also can be achieved chemically by catalytic hydrogenation in the usual manner (for example, under about one atmosphere of hydrogen in methanol) using a soluble rhodium catalyst with chiral phosphine ligands, such as (1,5-cyclooctadiene)-bis-(o-anisylcyclohexylmethylphosphine) rhodium (I) tetrafluoroborate in the presence of one equivalent of organic base, such as triethylamine.

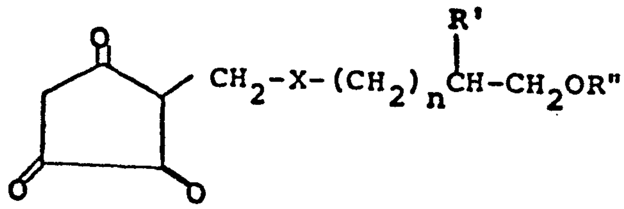

(86)

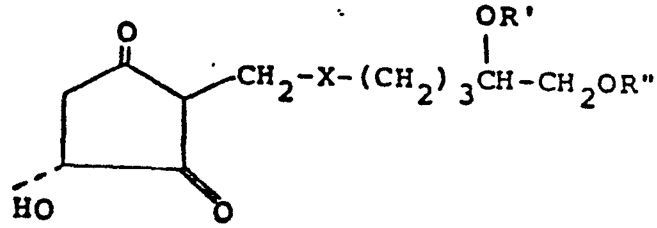

(87)

Procedures for the preparation of the requisite cyclopentanetriones are well-established in the art and generally involve the treatment of an ω-1-oxo long chain ester with methyl or ethyl oxalate and a base such as sodium methoxide in methanol, followed by treatment with hydrochloric acid in aqueous methanol. See J. Kutsube and M. Matsui, Agr. Biol. Chem., 33 1078 (1969); P. Collins, C.J. Jung and R. Pappo, Israel Journal of Chemistry 6, 839 (1968); R. Pappo, P. Collins and C. Jung, Ann, N.Y. Acad. Sci., 180, 64 (1971); C. J. Sih, et al., J.A.C.S., 95, 1676 (1973) (see reference 7); and J.B. Heather, et al., Tetrahedron Letters, 2313 (1973) for pertinent background literature.

It is also possible to resolve the 4-hydroxycyclopentone racemate (88) by microbiological means. Thus, treatment of the 4-O-alkanoyl or aroyl derivatives (89), $R_{18}$ = aryl or alkyl) of racemate (88) (preferably the 4-O-acetyl and 4-O-propionyl derivatives) with an appropriate microorganism, preferably a Saccharomyces species e.g., 1375-143, affords preferential de-O-acylation of the 4(R)-enantiomer to give (190), which is then separated from the unreacted 4(S)-O-acyl enantiomer (191) by chromatographic procedures. After separation, mild hydrolysis yields the 4(S) hydroxycyclopentenone (192). [See N. J. Marsheck and M. Miyano, Biochima et Biphysica Act, 316, 363 (1973) for related examples].

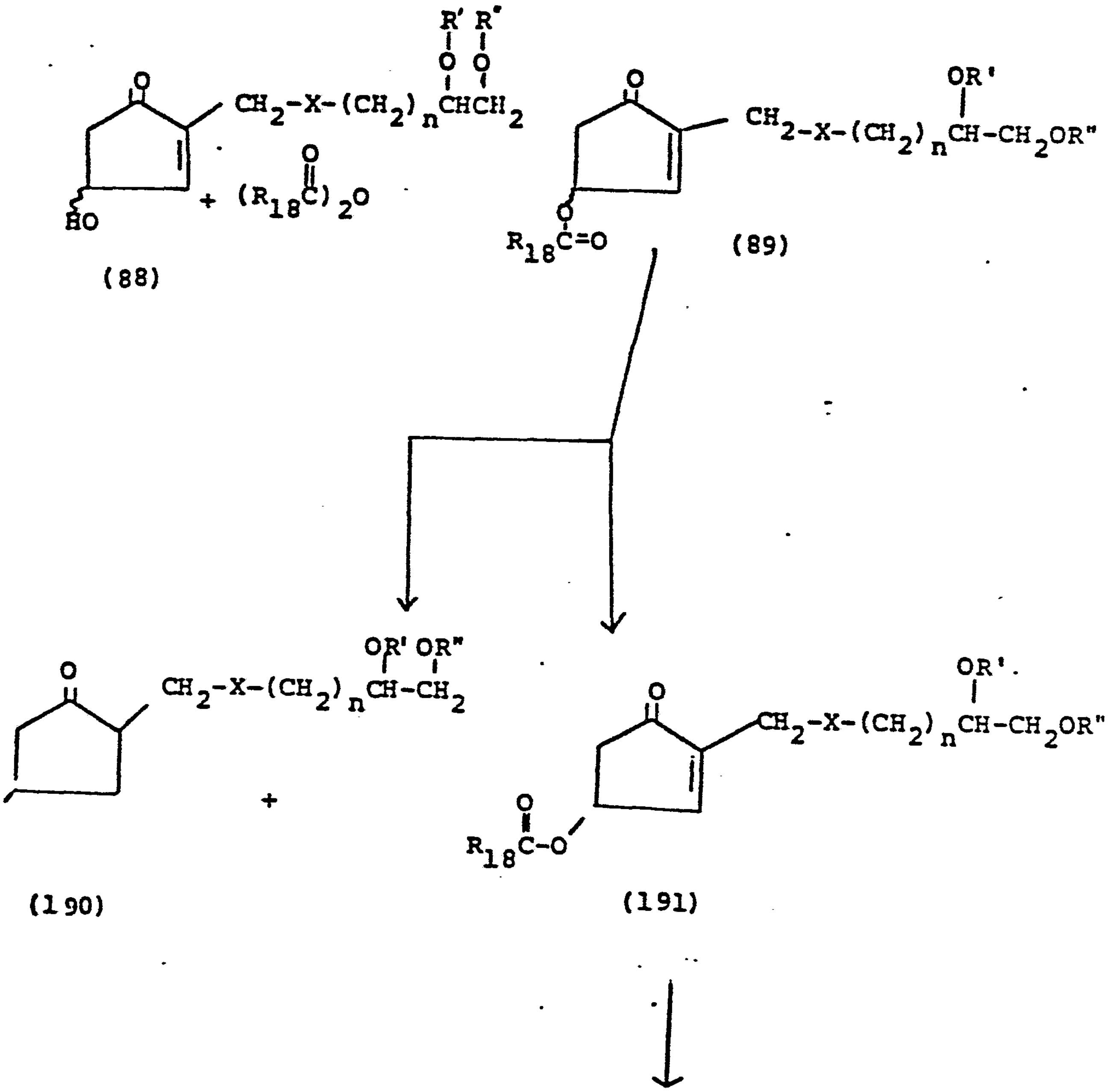

(88)

(89)

(190)

(191)

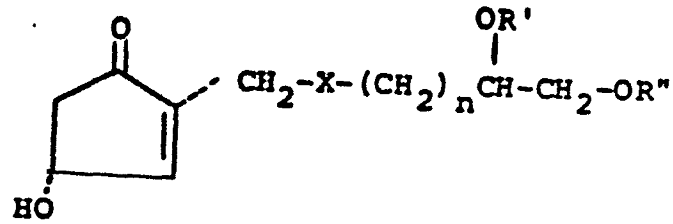

(192)

It is also possible to prepare the individual 4-hydroxycyclopentenones (190) and (192) directly by selective microbial hydroxylations of the corresponding 4-unsubstituted cyclopentenone (193). For example, with <u>Aspergillus</u> <u>niger</u> ATCC 9142; a selective 4(R) -hydroxylation of [(148, Z = $(CH_2)_6$] has been reported; see S. Kurozumi, T. Tora and S. Ishimoto, Tetrahedron Letters, 4959 (1973). Other microorganisms can also accomplish this hydroxylation.

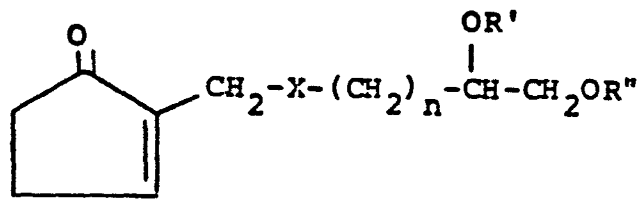

(193)

The ring system of the novel compounds of this invention allows them to be characterized as PGE-type compounds:

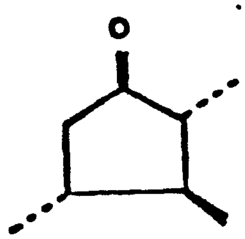

PGE-Type

The novel compounds of this invention possess the pharmacological activity described below as associated with the PGE prostaglandin type.

The known PGE compounds are all potent in causing multiple biological responses even at low doses. For example, $PGE_1$ and $PGE_2$ are extremely potent in causing vasodepression and smooth muscle stimulation, and also are potent as antilipolytic agents. Moreover, for many applications, these known prostaglandins have an inconviently short duration of biological activity. In striking contrast, the novel prostaglandin analogs of this invention are substantially more specific with regard to potency in causing prostaglandin-like biological responses, and/or having a substantially, longer duration of biological activity. Therefore, each of these novel prostaglandin analogs is surprisingly and unexpectedly more useful than one of the corresponding above-mentioned known prostaglandins for at least one of the pharmacological purposes indicated below for the latter, either because it has a different and narrower spectrum of biological activity than the known prostaglandins, and therefore is more specific in its activity and causes smaller and fewer undesired side effects than the known prostaglandins, or because of its prolonged activity, fewer and smaller doeses of the novel prostaglandin analog can frequently be used to attain the

desired result.

11-deoxy-PGE type compounds are additionally selective in that they are at most relatively very weak stimulants of smooth muscle. The 11-deoxy PGE compounds have a further advantage in that they are much more stable and have a longer "shelf-life" than the corresponding 11-hydroxy derivatives as described more fully hereinbelow.

Another advantage of the novel compounds of this invention, compared with the known prostaglandins, is that these novel compounds are administered effectively, orally, sublingually, intravaginally, buccally, or rectally, in addition to the usual intravenous, intramuscular, or subcutaneous injection or infusion methods indicated above for the uses of the known prostaglandins. These qualities are advantageous because they facilitate maintaining uniform levels of these compounds in the body with fewer, shorter, or smaller doeses and make possible self-administration by the patient.

$PGE_1$, $PGE_2$ and dihydro-$PGE_1$, and their esters and pharmacologically acceptable salts, are extremely potent in causing various biological responses. For that reason, these compounds are useful for pharmacological purposes. See, for example, Bergstron, et al., Pharmacol. Rev. 20, 1 (1968), and references cited therein. A few of those biological responses are systemic arterial blood pressure

lowering in the case of the PGE, compounds as measured, for example, in anesthetized (phenobarbital sodium) pentolinium--treated rats with indwelling aortic and right heart cannulas; pressor activity, similarly measured, for the PGF compounds; stimulation of smooth muscle as shown, for example, by tests on strips of guinea pig ileum, rabbit duodenum, or gerbil colon; potentiation of other smooth muscle stimulants; antilipolytic activity as shown by antagonism of epinephrine-induced mobilization of free fatty acids or inhibition of the spontaneous release of glycerol from isolated rat fat pads; inhibition of gastric secretion in the case of the PGE as shown in dogs with secretion stimulated by food or histamine infusion; activity on the central nervous system; decrease of blood platelet adhesiveness in the case of PGE, as shown by platelet-to-glass adhesiveness, and inhibition of blood platelet aggregation and thrombus formation induced by various physical stimuli, e.g., arterial injury and various biochemical stimuli, e.g. ADP, ATP, serotonin, thrombin and collagen; and in the case of the PGE compounds, stimulation of epidermal proliferation and keratinization as shown when applied in culture to embryonic chick and rat skin segments.

Because of these biological responses, the known PGE prostaglandins are useful to study, prevent, control, or alleviate a wide variety of disease and undesirable

physiological conditions in birds and mammals, including humans, useful domestic animals, pets, and zoological specimens, and in laboratory animals, for example, mice, rats, rabbits, and monkeys.

For example, PGE compounds, are useful in mammals, including man, as nasal decongestants. For this purpose, the compounds are used in a dose range of about 10 g to about 10 mg per ml of a pharmacologically suitable liquid vehicle or as an aerosol spray, both for topical application.

PGE compounds are also useful in mammals, including man and certain useful animals, e.g., dogs and pigs, to reduce and control excessive gastric secretion, thereby reducing or avoiding gastric erosion or gastrointestinal ulcer formation, and accelerating the healing of such ulcers already present in the gastrointestinal tract. For this purpose, the compounds are injected or infused intravenously, subcutaneously, or intramuscularly in an infusion dose range of about 0.1 g to about 500 g per kg of body weight per minute, or in a total daily dose by injection or infusion in the range of about 0.1 mg to about 20 mg per kg of body weight per day, the exact dose depending on the age, weight, and condition of the patient or animal, and on the frequency and route of administration. These compounds may also be useful in conjunction with

various non-steroidal anti-inflammatory agents, such as aspirin, phenylbutazone, indomethacin and the like, to minimize the well-known ulcerogenic effects of the latter.

$PGE_1$ compounds are useful whenever it is desired to inhibit platelet aggregation, to reduce the adhesive character of platelets, and to remove or prevent the formation of thrombi in mammals, including man, rabbits, and rats. For example, these compounds are useful in treatment and prevention of myocardial infarcts, to treat and prevent post-operative thrombosis. For these purposes, these compounds are administered systemically, e.g., intravenously, subcutaneously, intramuscularly, and in the form of sterile implants for prolonged action. For rapid response, especially in emergency situations, the intravenous route of administration is preferred. Doses in the range of about 0.005 mg to about 20 mg per kg of body weight per day are used, the exact dose depending on the age, weight, and condition of the patient or animal, and on the frequency and route of administration.

11α-Hydroxy-PGE compounds are extremely potent in causing stimulation of smooth muscle, and are also highly active in potentiating other known smooth muscle stimulators, for example, oxytocic agents, e.g., oxytocin, and the various ergot alkaloids including derivatives and analogs thereof. Therefore $PGE_2$, for example, is useful in place of or in combination with less than usual amounts of these known smooth

muscle stimulators, for example to relieve the symptons of paralytic ileus, or to control or prevent uterine bleeding after abortion or delivery, to aid in expulsion of the placenta, and during the puerperium. For the latter purpose, the PGE compound is administered by intravenous infusion immediately after abortion or delivery at a dose in the range about 0.01 g to about 50 g per kg of body weight per minute until the desired effect is obtained. Subsequent doses are given by intravenous, subcutaneous, or intramuscular injection or infusion during puerperium in the range of 0.01 mg to 2 mg per kg of body weight per day, the exact dose depending on the age, weight, and condition of the patient or animal.

PGE compounds are also useful as hypotensive agents to reduce blood pressure in mammals including man. For this purpose, the compounds are administered by intravenous infusion at the rate about 0.01 g to about 50 g per kg of body weight per minute, or in a single or multiple doses of about 25 g to 2500 g per kg of body weight total per day.

In addition, PGE type prostaglandins compounds are useful in place of oxytocin to induce labor in pregnant female animals, including man, cows, sheep, pigs, at or near term or in pregnant animals with intrauterine death of the fetus from about 20 weeks to term. For this purpose, the

compound is infused intravenously at a dose 0.01  g to 50  g per kg of body weight per minute until or near the termination of the second stage of labor, i.e., expulsion of the fetus. The compounds are especially useful when the female is one or more weeks postmature and natural labor has not started, or 12 to 60 hours after the membranes have ruptured and natural labor has not yet started.

PGE compounds are also useful for controlling the reproductive cycle in ovulating female mammals, including humans and other animals.

The novel PGE compounds of this invention are also useful as bronchodialtors for the treatment of asthma and chronic bronchitis. As such they may be conveniently administrered by inhalation of aerosol sprays prepared in a dose range of about 10  g to about 10 mg/ml of a pharmacologically suitable liquid vehicle. Relative to the natural prostaglandins, the PGE compounds in particular have the significant advantage of inducing prolonged effects.

The compounds of this invention are useful as vasodilators whose activity may be restricted to the site of application by control of the dose applied. Compositions containing the compounds of this invention may be applied topically and by local injection to increase peripheral circulation or to treat peripheral vascular disorders of the

arteriospastic and occlussive types. Compositions for topical administration containing prostaglandins similar to those of this invention are disclosed by application Serial No. 934,199 filed August 16, 1978, now U.S. Patent No. 4,254,145. The compounds of this invention may be employed as vasodilators for topical application in the compositions and dosages described by the above-cited applications. Typically topical preparations contain from about .3 to 20% by W/W of the compounds of this invention in a pharmaceutical carrier, and preferably from about 1% to 5% by W/W of the prostaglandin.

The compounds of this invention may also be administered topically or by local injection to lower the systemic blood pressure of man and warm blooded animals. Systemic action is obtained by the use of an effective dosage greater than that used to induce localized action at the site of application. The effective dosage of the compounds of this invention administered topically in a pharmaceutical carrier for systemic effect is about .1 mg. to 10 mg. per kg. of body weight per day. Pharmaceutical carriers and compositions which may be employed are disclosed by application Serial No. 934,199, filed August 16, 1978; U.S. Patent No. 4,254,145.

This invention will be described in greater detail with reference to the following preparations and examples.

Preparation 1

2-Hydroxynonanedioic Acid

A stirred mixture of 295 g. of dimethyl 2-bromononanedioate, [Acta. Chim. Acad. Sci. Hung., 46, 85 (1965)], 371 g. of anhydrous sodium carbonate, and 3 liters of water is heated at reflux for 22 hours. The solution which forms is cooled and acidified with 600 ml. of 12N hydrochloric acid. The mixture is saturated with sodium chloride and extracted with ethyl acetate. The extract is washed with brine, dried over magnesium sulfate, and concentrated to give 184 g. of white solid, m.p. 86-92°C.

Preparation 2

4-(6-Carboxyhexyl)-2,2-dimethyl-5-oxo-1,3-dioxolane

A stirred mixture of 10.2 g. of 2-hydroxynonanedioic acid, 50 ml. of acetone, 75 ml. of petroleum ether (b.p. 30--55°C.), and 0.25 ml. of sulfuric acid is heated at reflux for 7 hours with azeotropic removal of water. The stirred mixture is treated with 0.59 g. of sodium carbonate and diluted with petroleum ether. The solution is decanted from solid and concentrated to give 9.4 g. of oil; pmr ($CDCl_3$) 1.58 and 1.63 (gem-dimethyl group).

Preparation 3

8-(2-Furoyl)-2-hydroxyoctanoic Acid

To a stirred, ice-cold solution of 83 g. of 4-(6-carboxyhexyl)-2,2-dimethyl-5-oxo-1,3-dioxolane in 340 ml. of dichloromethane is added 48 ml. of trifluoroacetic anhydride during 5 minutes. The solution is warmed to 20°C. during 10 minutes, recooled to 0°C., and then is treated with 122 ml. of furan during 10 minutes. The resulting solution is stirred at room temperature for 16 hours. The solution is treated with 68 ml. of concentrated sulfuric acid during 15 minutes, and then is heated at reflux temperature for 18 hours. The solution is cooled, saturated with sodium chloride, and extracted with ethyl acetate. The extract is washed with brine, dried over magnesium sulfate, and concentrated. The resulting crude product is purified by chromatography on silica gel with ether progressively enriched in methanol to give an oil; pmr ($CDCl_3$) 5.94 (multiplet) and 7.22 (broad singlet).

Preparation 6

2-(7,8-Bistrimethylsilyloxyoctyl)-4--trimethylsilyloxycyclopent-2-en-1-one

To a stirred, ice-cold solution of 8.2 g. of 8-(4-hydroxycyclopent-2-en-1-on-2-yl)-1,2-octanediol in 100 ml. of pyridine is added 26 ml. of hexamethyldisilazane followed by 13 ml. of chlorotrimethylsilane. The resulting mixture is stirred at room temperature for 4 hours. Solvent and excess reagents are removed by

evaporation under vacuum, and the residue is treated with petroleum ether and filtered through celite. The residue obtained after solvent evaporation of the filtrate is subjected to Kugelrohr distillation at 160°C. to provide a colorless liquid.

Preparation 7

2,2-Dimethyl-4-(4-p-toluenesulfonyloxybutyl)-1,3-dioxolane

To a stirred, ice-cold solution of 6.97 g. of 2,2-dimethyl-4-(4-hydroxybutyl)-1,3-dioxolane [Chem. Abstr., 67, 32617f (1967)] in 40 ml. of pyridine is added 9.2 g. of p-toluenesulfonyl chloride. The mixture is kept at 0-5°C. overnight, diluted with ice water, and extracted with ether. The extract is washed successively with water, sodium bicarbonate, water and brine. The extract is dried over magnesium sulfate and concentrated to give a syrup; pmr($CDCl_3$) 2.50 (singlet).

Preparation 8

4-(4-Bromobutyl)-2,2-dimethyl-1,3-dioxolane

To a stirred solution of 220 g. of 2,2-dimethyl-4-(4-p-toluenesulfonyloxybutyl)-1,3-dioxolane in 800 ml. of dimethylformamide at 0°C. is added 115 g. of anhydrous lithium bromide in portions during 5 minutes. The resulting mixture is stirred at ambient temperature for 22 hours, diluted with water, and extracted with ether. The extract is washed with sodium bicarbonate solution, water,

and brine; dried over magnesium sulfate; and concentrated. The residue is distilled to give a colorless liquid, b.p. 71-74°C. (0.2 mm).

Preparation 9

(5,6-Dihydroxyhexyl)triphenylphosphonium bromide

A stirred solution of 125 g. of 4-(4-bromobutyl)-2,2-dimethyl-1,3-dioxolane, 154 g. of triphenylphosphine, 800 ml. of acetonitrile, and 270 ml. of acetone is heated at reflux for 69 hours. The solvents are removed under vacuum, and the residue is treated with 250 ml. of chloroform and 15 ml. of water. The resulting mixture is stirred vigorously for 30 minutes. The solvents are removed with the aid of benzene chaser, and the solid product is washed with ether and dried under vacuum to give white crystals, m.p. 152-170°C.

Preparation 10

2,5-Dihydro-2,5-dimethoxy-2-
-(8',9'-dihydroxy-3'-cis-octenyl) furan

A solution of 37.2 g. of 2,5-dihydro-2,5-dimethoxy-2-(3'-oxopropyl) furan in 150 ml. of dimethylsulfoxide is added during 20 minutes at 18-20°C. to the Wittig reagent prepared from 200 ml. of 1 M. dimsyl sodium in dimethylsulfoxide and 96 g. of (5,6-dihydroxyhexyl) triphenylphosphonium bromide in 300 ml. of dimethylsulfoxide. The resulting solution is stirred at 25°C. for 17 hours. The

solvent is removed under vacuum, and the residue is treated with brine and extracted with ethyl acetate. The extract is washed with water and brine, dried over magnesium sulfate, and concentrated to give an oil, pmr ($CDCl_3$) 5.5. (multiplet).

Preparation 11

2-(7',8'-Dihydroxy-2'-*cis*-octenyl)--4-hydroxycyclopent-2-en-1-one

To a well-agitated and cooled solution of 100 g. of crude 2,5-dihydro-2,5-dimethoxy-2-(8',9'-dihydroxy-3'--*cis*-octenyl) furan in 1500 ml. of chloroform is added 2400 g. of silica gel and 250 ml. of water. After evaporation of 750 ml. of chloroform the resulting slurry is kept at 25°C. for 6 days, and then eluted with 8 liters of 4:1 ethyl acetate-methanol.

The eluate is concentrated, and the residue is dissolved in 900 ml. of dioxane and 600 ml. of water. To the stirred solution is added 100 mg. of hydroquinone and 42 ml. of sulfuric acid dropwise during 10 minutes. The resulting solution is heated under reflux for 21 hours, cooled, saturated with sodium chloride, and extracted with ethyl acetate. The extract is washed with brine, dried over magnesium sulfate, and concentrated. The residue is subjected to column chromatography on silica gel with ether progressively enriched in methanol to give an oil;

pmr 5.97 (multiplet) and 7.22 (broad singlet).

Preparation 12

2-(7',8'-Bistrimethylsilyloxy)-2'-cis-octenyl)--4-trimethylsilyloxycyclopent-2-en-1-one

To a stirred, ice-cold solution of 4.8 g. of 2-(7',8'-dihydroxy-2'cis-octenyl)-4-hydroxycyclopent-2-en-1-one in 60 ml. of pyridine is added successively 15 ml. of hexamethyldisilazane and 7.6 ml. of chlorotrimethylsilane. The resulting mixture is stirred at ambient temperature for 5 hours. The excess reagents and solvent are removed under vacuum, and the residue is slurried with petroleum ether and filtered through celite. The filtrate is concentrated to give an amber liquid.

The following examples are illustrated of the preparation of various $E_1$ and $E_2$-series prostaglandins all bearing the 1-hydroxy-1-hydroxymethyl moiety of the present invention.

By reaction of the cyclopentanetriones intermediates of Examples 6 and 12 with a vinylstannyl or vinyl iodide beta chain intermediate, the 1-hydroxymethyl-1-hydroxy prostaglandins set forth in the following examples are prepaid.

Example 1

11 α, 16-Dihvdroxv-1-hydroxymethyl-16--methyl--,9--oxo-13-trans-prosten-1-ol

A stirred solution of 5.04 g. of 1-tri-n-butylstannyl-4-methyl-4-trimethylsiloxy-1-trans-octen in 8.0 ml. of tetrahydrofuran is treated with 4.0 ml. of 2.0 M. n-butyllithium in hexane at -78°C. The resulting solution is stirred at -40°C. for 2 hours, recooled to -78°C., and treated with a solution prepared from 1.16 g. of copper pentyne, 3.2 ml. of hexamethylphosphorous triamide, and 20 ml. of ether. The resulting solution is stirred at -78°C. for one hour and then treated with a solution of 1.84 g. of 2-(7',8'-bistrimethylsilyloxyoctyl)-4-trimethylsilyloxycyclopent-2-en-1-one (Preparation 6) in 10 ml. of ether during 5 minutes. The resulting solution is stirred at -40°C. for 90 minutes, recooled to -78°C., and quenched with a solution of 1.0 ml. of acetic acid in 20 ml. of ether.

The mixture is diluted to 150 ml. ether and poured into a stirred, ice-cold mixture of 40 ml. of saturated ammonium chloride solution and 40 ml. of 1.0 N hydrochloric acid. The ether layer is washed successively with 2.0 N hydrochloric acid, water, and brine; dried over magnesium sulfate; and concentrated.

The residue is treated with a solution prepared from 32 ml. of acetic acid, 16 ml. of tetrahydrofuran, and 8 ml. of water, and the resulting mixture is stirred at 40°C. for 60 minutes. The mixture is cooled and

partitioned with ethyl acetate and half-saturated brine. The organic layer is washed with brine, dried over magnesium sulfate, and concentrated to give a mixture of oil and liquid tetrabutylstannane. These materials are separated by successive elution through silica gel with hexane and ethyl acetate. The ethyl acetate eluate is concentrated, and the residue is subjected to chromatography on silica gel to provide an oil.

## Example 2

### 11α16-Dihydroxy-1-hydroxymethyl-16--methyl-9-oxo-5-*cis*-13-*trans*-prostadien-1-ol

A stirred solution of 2.52 g. of (4-methyl-4--trimethylsiloxy-1-*trans*-octenyl)tributylstannane in 5.0 ml. of tetrahydrofuran is treated with 2.1 ml. of 2.4 M. *n*-butyllithium in hexane at -78°C. The solution is stirred at -40°C. for 1.5 hours, warmed to -30°C. during 15 minutes, and recooled to -78°C. The stirred solution is treated with a solution prepared from 0.66 g. of copper pentyne, 2.5 ml. of tri-*n*-butylphosphine, and 6.5 ml. of ether. The solution is stirred at -78°C. for 90 minutes and then treated with a solution of 1.83 g. of 2-(7',8'-trimethylsilyloxy-2-*cis*-octenyl)-4-trimethylsilyloxycyclopent-2-en-1-one (Preparation 12) in 7.5 ml. of ether during 10 minutes. The solution is stirred at -45° to -40°C for 60 minutes and at -40° to -35°C for 45 minutes.

The solution is recooled to -78°C and quenched with a solution of 0.60 ml. of acetic acid in 10 ml. of ether. The solution is poured into a stirred mixture of ether and saturated ammonium chloride. The organic layer is separated and washed successively with water and brine, dried over magnesium sulfate, and concentrated.

The residue is treated with 32 ml. of acetic acid, 16 ml. of tetrahydrofuran, and 8 ml. of water, and the resulting solution is stirred at 40°C. for one hour. The solution is partitioned with half-saturated brine and ethyl acetate. The organic layer is washed with brine, dried over magnesium sulfate, and concentrated. The residue is partitioned between n-heptane and 50:1 methanol-water.

The methanol phase is concentrated to give the crude product which is subjected to column chromatography on silica gel to give an oil.

Example 3

11α,16-Dihydroxy-1-hydroxymethyl-16--vinyl-9-oxo-5-cis-13-trans-prostadien-1-ol

A stirred solution of 3.54 g. of (4-trimethylsiloxy-4-vinyl-1-trans-octenyl)tributylstannane in 5.0 ml. of tetrahydrofuran is treated with 3.43 ml. of 2.2 M. n-butyllithium in hexane at -78°C. The resulting solution is allowed to warm to -23°C. over a

period of 2 hours, is recooled to -78°C., and then treated with a solution prepared from 0.90 g. of copper pentyne, 2.39 ml. of hexamethylphosphoroustriamide, and 5 ml. of tetrahydrofuran. The resulting solution is stirred at -78°C. for 70 minutes and then treated with a solution of 2.41 g. of 2-(7',8'-bistrimethylsilyloxy-2--cis-octenyl)-4-trimethylsilyloxycyclopent-2-en-1-one (Preparation 12) in 10 ml. of tetrahydrofuran during 5 minutes. The resulting solution is stirred at -78°C for 20 minutes, at -30°C for 90 minutes, and is recooled to -45°C and poured into a stirred, ice-cold mixture of 200 ml. of saturated ammonium chloride and 100 ml of ether. The ether phase is separated and washed successively with cold 5% hydrochloric acid and brine, dried over magnesium sulfate, and concentrated.

The resulting oil is treated with a mixture of 50 ml. of acetic acid, 25 ml. of tetrahydrofuran, and 12.5 ml. of water. The resulting mixture is stirred at 25°C. for 60 minutes diluted with toluene, and evaporated under vacuum. The residue is washed with hexane to remove tetrabutylstannane by decantation, and the oil remaining is subjected to chromatography on silica gel to give an oil.

Example 4 - 20

The prostaglandin of Examples 4 through 20

were prepared from the listed cyclopentenone and vinyl iodide or vinyl tin intermediate in accordance with the procedures of Examples 1 through 3.

Example 4

1-trans-IODO--

3-TRIPHENYLMETHOXY-1-OCTENE

CYCLOPENTENONE

2-(7,8-bis-TRIMETHYLSILYLOXYOCT-2-cis-ENYL)

-4-TRIMETHYLSILOXYCYCLOPENT-2-EN-1-ONE

PRODUCT PROSTAGLANDIN OF THE PGE2 SERIES

dl-1,11α,15α-TRIHYDROXY-1-HYDROXYMETHYL-9-OXO--

-5-cis-13-trans PROSTADIENE

Example 5

1-trans-IODO-3-METHYL-3-TRIMETHYLSILYLOXY-1-OCTENE

CYCLOPENTENONE

2-(7,8-bis-TRIMETHYLSILYLOXYOCT-2-cis-ENYL)

-4-TRIMETHYLSILOXYCYCLOPENT-2-EN-1-ONE

PRODUCT PROSTAGELANDIN OF THE PGE2 SERIES

dl-1,11α,15α-TRIHYDROXY-1-HYDROXYMETHYL-9-OXO-15-METHYL--5-cis-13-trans PROSTADIENE

Example 6

1-trans-IODO--

3-TRIPHENYLMETHOXY-1-OCTENE

CYCLOPENTENONE

2-(7,8-bis-TRIMETHYLSILYLOXYOCT-2-cis-ENYL)

-4R-TRIMETHYLSILOXYCYCLOPENT-2-EN-1-ONE

PRODUCT PROSTAGLANDIN OF THE PGE2 SERIES

nat-1,11α,15α-TRIHYDROXY-1-HYDROXYMETHYL-9-OXO--

-5-cis-13-trans PROSTADIENE

Example 7

1-trans-IODO-3-METHYL-

3-TRIMETHYLSILYLOCY-1-OCTENE

CYCLOPENTENONE

2-(7,8-bis-TRIMETHYLSILYLOXYOCT-2-cis-ENLY)

-4R-TRIMETHYLSILOXYCYCLOPENT-2-EN-1-ONE

PRODUCT PROSTAGELANDIN OF THE PGE2 SERIES

nat-1,11α,15α-TRIHYDROXY-1-HYDROXYMETHYL-9-OXO-15-METHYL--5-cis-13-trans PROSTRADIENE

Example 8

1-trans-IODO--

4-TRIPHENYLMETHOXY-1-OCTENE

CYCLOPENTENONE

2-(7,8-bis-TRIMETHYLSILYLOXYOCT-2-cis-ENYL)

-4R-TRIMETHYLSILOXYCYCLOPENT-2-EN-1-ONE

PRODUCT PROSTAGLANDIN OF THE PGE2 SERIES

nat-1,11α,16-TRIHYDROXY-1-HYDROXYMETHYL-9-OXO--

-5-cis-13-trans PROSTADIENE

Example 9

1-trans-tri-n-BUTYLSTANNYL-4-METHYL-

4-TRIMETHYLSILYLOXY-1-OCTENE

CYCLOPENTENONE

2-(7,8-bis-TRIMETHYLSILYLOXYOCT-2-cis-ENYL)

-4R-TRIMETHYLSILOXYCYCLOPENT-2-EN-1-ONE

PRODUCT PROSTAGLANDIN OF THE PGE2 SERIES

nat-1,11α,16-TRIHYDROXY-1-HYDROXYMETHYL-9-OXO-16-METHYL--5-cis-13-trans PROSTADIENE

Example 10

1-trans-tri-n-BUTYLSTANNYL-4-VINYL-

4-TRIMETHYLSILYLOXY-1-OCTENE

CYCLOPENTENONE

2-(7,8-bis-TRIMETHYLSILYLOXYOCT-2-cis-ENYL)

-4R-TRIMETHYLSILOXYCYCLOPENT-2-EN-1-ONE

PRODUCT PROSTAGLANDIN OF THE PGE2 SERIES

nat-1,11α,16-TRIHYDROXY-1-HYDROXYMETHYL-9-OXO-16-VINYL--5-cis-13-trans PROSTADIENE

Example 11

1-trans-IODO--

4-TRIPHENYLMETHOXY-1-OCTENE

CYCLOPENTENONE

2-(7,8-bis-TRIMETHYLSILYLOXYOCT-2-cis-ENYL)

-4-TRIMETHYLSILOXYCYCLOPENT-2-EN-1-ONE

PRODUCT PROSTAGLANDIN OF THE PGE2 SERIES

dl-1,11α,16-TRIHYDROXY-1-HYDROXYMETHYL-9-OXO--

-5-cis-13-trans PROSTADIENE

Example 12

1-trans-IODO-

4-TRIPHENYLMETHOXY-1-OCTENE

CYCLOPENTENONE

2-(7,8-bis-TRIMETHYLSILYLOXYOCTYL)

-4-TRIMETHYLSILOXYCYCLOPENT-2-EN-1-ONE

PRODUCT PROSTAGLANDIN OF THE PGE1 SERIES

dl-1,11α,16-TRIHYDROXY-1-HYDROXYMETHYL-9-OXO--13-trans PROSTENE

Example 13

1-trans-tri-n-BUTYLSTANNYL-4-VINYL-

4-TRIMETHYLSILYLOXY-1-OCTENE

CYCLOPENTENONE

2-(7,8-bis-TRIMETHYLSILYLOXYOCTYL)

-4-TRIMETHYLSILOXYCYCLOPENT-2-EN-1-ONE

PRODUCT PROSTAGLANDIN OF THE PGE1 SERIES

dl,1,11α,16-TRIHYDROXY-1-HYDROXYMETHYL-9-OXO-16-VINYL--13-trans PROSTENE

Example 14

1-trans-tri-n-BUTYLSTANNYL-4-VINYL-

4-TRIMETHYLSILYLOXY-1-OCTENE

CYCLOPENTENONE

2-(7,8-bis-TRIMETHYLSILYLOXYOCTYL)

-4R-TRIMETHYLSILOXYCYCLOPENT-2-EN-1-ONE

PRODUCT PROSTAGLANDIN OF THE PGE1 SERIES

nat-1,11α,16-TRIHYDROXY-1-HYDROXYMETHYL-9-OXO-16-VINYL-
-13-trans PROSTENE

Example 15

1-trans-IODO-
4-TRIPHENYLMETHOXY-1-OCTENE

CYCLOPENTENONE

2-(7,8-bis-TRIMETHYLSILYLOXYOCTYL)
-4R-TRIMETHYLSILOXYCYCLOPENT-2-EN-1-ONE

PRODUCT PROSTAGLANDIN OF THE PGE1 SERIES

nat-1,11α,16-TRIHYDROXY-1-HYDROXYMETHYL-9-OXO-
-13-trans PROSTENE

Example 16

1-trans-tri-n-BUTYLSTANNYL-4-METHYL-
4-TRIMETHYLSILYLOXY-1-OCTENE

\- CYCLOPENTENONE

2-(7,8-bis-TRIMETHYLSILYLOXYOCTYL)
-4R-TRIMETHYLSILOXYCYCLOPENT-2-EN-1-ONE

PRODUCT PROSTAGLANDIN OF THE PGE1 SERIES

nat-1,11α,16-TRIHYDROXY-1-HYDROXYMETHYL-9-OXO-16-METHYL-
-13-trans PROSTENE

Example 17

1-trans-IODO-
3-TRIPHENYLMETHOXY-1-OCTENE

CYCLOPENTENONE

2-(7,8-bis-TRIMETHYLSILYLOXYOCTYL)

-4-TRIMETHYLSILOXYCYCLOPENT-2-EN-1-ONE

PRODUCT PROSTAGLANDIN OF THE PGE1 SERIES

dl-1,11α,15α-TRIHYDROXY-1-HYDROXYMETHYL-9-OXO--13-trans PROSTENE

Example 18

1-trans-IODO-3-METHYL-3-TRIMETHYLSILYLOXY-1-OCTENE

CYCLOPENTENONE

2-(7,8-bis-TRIMETHYLSILYLOXYOCTYL)

-4-TRIMETHYLSILOXYCYCLOPENT-2-EN-1-ONE

PRODUCT PROSTAGLANDIN OF THE PGE1 SERIES

dl-1,11α,15α-TRIHYDROXY-1-HYDROXYMETHYL-9-OXO-15-METHYL--13-trans PROSTENE

Example 19

1-trans-IODO-

3-TRIPHENYLMETHOXY-1-OCTENE

CYCLOPENTENONE

2-(7,8-bis-TRIMETHYLSILYLOXYOCTYL)

-4R-TRIMETHYLSILOXYCYCLOPENT-2-EN-1-ONE

PRODUCT PROSTAGLANDIN OF THE PGE1 SERIES

nat-1,11α,15α-TRIHYDROXY-1-HYDROXYMETHYL-9-OXO--13-trans PROSTENE

Example 20

1-trans-IODO-3-METHYL-3-TRIMETHYLSILYLOXY-1-OCTENE

CYCLOPENTENONE

2-(7,8-bis-TRIMETHYLSILYLOXYOCTYL)

-4R-TRIMETHYLSILOXYCYCLOPENT-2-EN-1-ONE

PRODUCT PROSTAGLANDIN OF THE PGE1 SERIES

nat-1,11α,15α-TRIHYDROXY-1-HYDROXYMETHYL-9-OXO-15-METHYL-

-13-trans PROSTENE

CLAIMS:

1. Compounds of the formula:

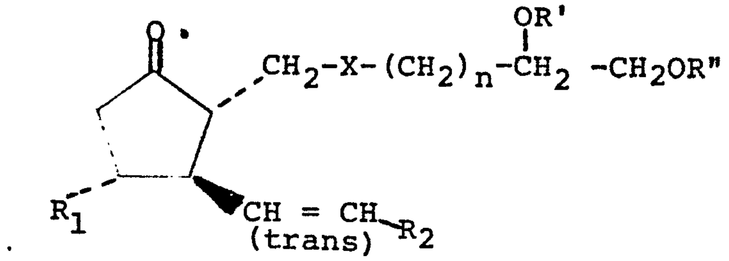

wherein n is the interger 3-5 inclusive; X is $CH_2CH_2$ or CH = CH (cis); $R_1$ is hydrogen or hydroxy, R' and R" are the same or different and are hydrogen, $C_1$ to $C_4$ alkyl, $C_1$ to $C_6$ alkanoyl or optionally substituted benzoyl, wherein said substituents on said benzoyl moiety are selected from the group $C_1$ to $C_4$ alkoxy, halo and trifluoromethyl; and $R_2$ is selected from the group consisting of

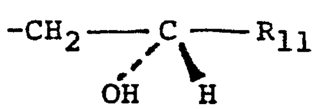, 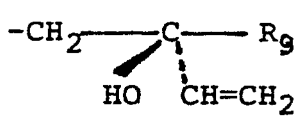, 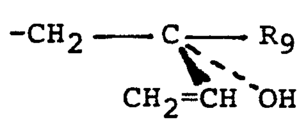

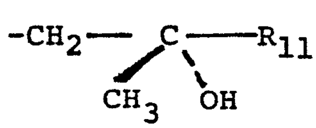, 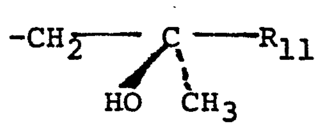, 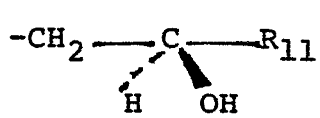

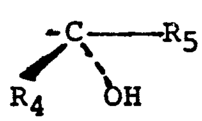 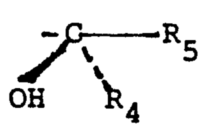

wherein $R_4$ is hydrogen or methyl; $R_5$ is selected from the group consisting of $C_4$-$C_7$ alkyl; $R_9$ is selected from the group consisting of $C_3$-$C_6$; $R_{11}$ is selected from the group consisting of $C_3$-$C_7$ alkyl; the racemic mixtures thereof and the individual optically active enantiomers thereof; and the pharmaceutically acceptable non-toxic salts thereof.

2. The compound according to Claim 1 wherein X is $CH_2$-$CH_2$ and n is 3.

3. The compound according to Claim 1 wherein X is CH = CH (trans) and n is 3.

4. The compound according to Claims 2 or 3 wherein $R_2$ is

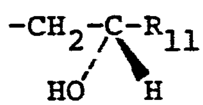

or

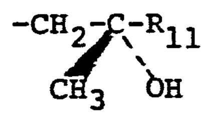

5. The compound according to Claims 2 or 3 wherein $R_2$ is 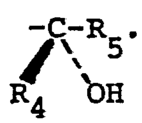.

6. The compound according to Claim 5 wherein $R_4$ is methyl and $R_5$ is a $C_5$-alkyl substituent.

7. The compound according to Claim 5 wherein $R_4$ is hydrogen and $R_5$ is a $C_5$-alkyl substituent.

8. The compound according to Claims 2 or 3 wherein $R_2$ is 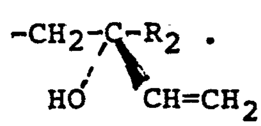.

9. The compound according to Claim 8 wherein $R_2$ is a $C_4$-alkyl substituent.

10. The compound according to Claim 1 selected from the group consisting of: dl- or nat-1,11α,16-trihydroxy-1-hydroxymethyl-9-oxo-16-methyl-13-trans prostene; dl- or nat-1,11α,16-trihydroxy-1-hydroxymethyl-9-oxo-16-methyl-5-cis-13-trans prostadiene; dl- or nat-1,11α,16-trihydroxy-1-hydroxymethyl-9-oxo-16-vinyl-5-cis-13-trans prostadiene; dl- or nat-1,11α,15α-trihydroxy-1-hydroxymethyl-9-oxo-5-cis-13-trans prostadiene; dl- or nat-1,11α,15α-trihydroxy-1-hydroxymethyl-9-oxo-15-methyl-5-cis-13-trans prostadiene; dl- or nat-1,11α,16-trihydroxy-1-hydroxymethyl-9-oxo-5-cis-13-trans prostadiene; dl- or nat-1,11α,16-trihydroxy-1-hydroxymethyl-9-oxo-13-trans prostene; dl- or nat-1,11α,16-trihydroxy-1-hydroxymethyl-9-oxo-16-vinyl-13-trans prostene; dl- or nat-1,11α,15α-trihydroxy-1-hydroxymethyl-9-oxo-13-trans prostene; or dl- or nat-1,11α,15α-trihydroxy-1-hydroxymethyl-9-oxo-15-methyl-13-trans prostene.

European Patent Office

# EUROPEAN SEARCH REPORT

0072872

Application number

EP 81 10 6499

## DOCUMENTS CONSIDERED TO BE RELEVANT

| Category | Citation of document with indication, where appropriate, of relevant passages | Relevant to claim |
|---|---|---|
| A | EP - A - 0 002 590 (AMERICAN CYANAMID COMPANY)<br>* claims *<br>-------- | 1 |

**CLASSIFICATION OF THE APPLICATION (Int. Cl. 3)**

C 07 C 177/00
A 61 K 31/557//
C 07 C 59/245
C 07 D 317/34
307/54
C 07 F 7/18
C 07 D 317/22
317/16
C 07 F 9/34
C 07 D 307/30
C 07 C 49/203
C 07 F 7/18

**TECHNICAL FIELDS SEARCHED (Int.Cl. 3)**

C 07 C 177/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 03.02.1982 | BERTE |

EPO Form 1503.1 06.78